# EUROPÄISCHE PATENTSCHRIFT

(11) **EP 1 265 889 B1**
(45) Veröffentlichungstag und Bekanntmachung des Hinweises auf die Patenterteilung: **16.06.2004**
(21) Anmeldenummer: 01921313.1
(22) Anmeldetag: 05.03.2001
(51) Int. Cl.: C07D 401/04, C07D 417/04, C07D 401/14, A61K 31/506, A61P 31/12

(54) **ARZNEIMITTEL GEGEN VIRALE ERKRANKUNGEN**
MEDICAMENTS AGAINST VIRAL DISEASES
AGENTS PHARMACEUTIQUES CONTRE DES MALADIES VIRALES

(30) Priorität: 16.03.2000 DE 10012823
(43) Veröffentlichungstag der Anmeldung: 18.12.2002
(73) Patentinhaber: Bayer HealthCare AG, 51368 Leverkusen (DE)
(72) Erfinder: GOLDMANN, Siegfried, 42327 Wuppertal (DE); STOLTEFUSS, Jürgen, 42781 Haan (DE); NIEWÖHNER, Ulrich, 42929 Wermelskirchen (DE); SCHLEMMER, Karl-Heinz, 42113 Wuppertal (DE); KELDENICH, Jörg, 42113 Wuppertal (DE); PAESSENS, Arnold, 42781 Haan (DE); GRAEF, Erwin, 78315 Radolfzell (DE); WEBER, Olaf, c/o Bayer Corp., West Haven, CT 06516-4175 (US); DERES, Karl, 53489 Sinzig (DE)
(86) Internationale Anmeldenummer: PCT/EP2001/002442
(87) Internationale Veröffentlichungsnummer: WO 2001/068640

(56) Entgegenhaltungen:
- WO-A-99/01438
- WO-A-99/54312
- WO-A-99/54326

## Beschreibung

Die vorliegende Erfindung betrifft neue 6-Aminoalkyl-dihydropyrimidine, Verfahren zu ihrer Herstellung sowie ihre Verwendung als Arzneimittel, insbesondere zur Behandlung und Prophylaxe von Hepatitis-B-Virus-Infektionen. Die Erfindung betrifft auch Kombinationen dieser Dihydropyrimidine mit anderen antiviralen Mitteln und gegebenenfalls Immunmodulatoren sowie Arzneimittel enthaltend diese Kombinationen, insbesondere zur Behandlung und Prophylaxe von HBV-Infektionen wie Hepatitis B.

Das Hepatitis-B-Virus gehört zur Familie der Hepadna-Viren. Es verursacht eine akute und/oder eine peristent-progrediente, chronische Erkrankung. Vielfältige andere klinische Manifestationen im Krankheitsbild werden durch das Hepatitis-B-Virus mitverursacht - insbesondere chronische Leberentzündung, Leberzirrhose und hepatozelluläres Karzinom. Weiterhin kann eine Koinfektion mit dem Hepatitis-Delta-Virus den Krankheitsverlauf negativ beeinflussen.

Die einzigen für die Behandlung chronischer Hepatitis zugelassenen Mittel sind Interferon und Lamivudin. Allerdings ist Interferon nur mäßig wirksam und hat unerwünschte Nebenwirkungen; Lamivudin ist zwar gut wirksam, aber unter Behandlung kommt es rasch zu einer Resistenzentwicklung, und nach Absetzen der Therapie erfolgt in den meisten Fällen ein Rebound-Effekt.

Aus der EP-PS 103 796 sind Dihydropyrimidine bekannt, denen eine den Kreislauf beeinflussende Wirkung zugeschrieben wird. Die WO 99/1438 betrifft Dihydropyrimidine, die sich für die Behandlung von cerebrovasculärer Ischämie und von Schmerz eignen sollen. Die WO 99/54312, 99/54326 und 99/54329 betreffen Dihydropyrimidine, die sich zur Behandlung und Prophylaxe von Hepatitis eignen.

Die vorliegende Erfindung betrifft Verbindungen der Formel 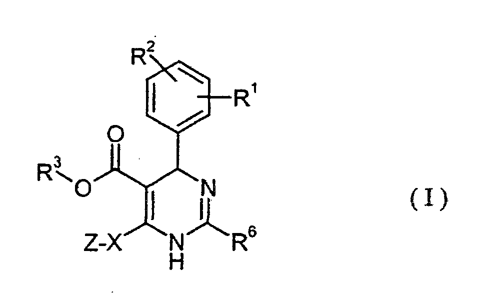 bzw. deren isomerer Form 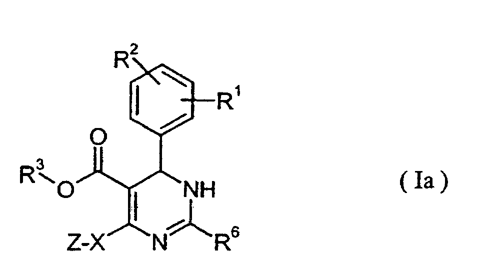 worin
- R¹, R²: unabhängig voneinander Wasserstoff, Fluor, Chlor oder Brom,
- R³: C₁-C₄-Alkyl,
- X: eine Methylen- oder Ethylengruppe,
- Z: NR⁴R⁵ oder Pyridyl,
- R⁴: C₁-C₄-Alkyl, das durch Hydroxy oder C₁-C₄-Alkoxycarbonyl substituiert sein kann, oder Benzyl,
- R⁵: C₁-C₄-Alkyl, das durch Hydroxy substituiert sein kann,
oder
- R⁴ und R⁵: gemeinsam mit dem Stickstoffatom, an das sie gebunden sind, einen Imidazolyl-, Triazolyl- oder Tetrazolyl-Ring oder einen Rest der Formel 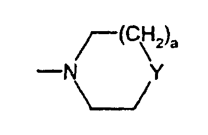 worin
a für Null oder 1 und
Y für CH₂, CH₂CH₂, -O- oder -S- stehen,
und
R⁶ Pyridyl, das ein- bis zweifach durch Fluor substituiert ist, oder Thiazolyl
bedeuten, und deren Salze.

Bevorzugt sind erfindungsgemäße Verbindungen der Formeln (I) bzw. (I a), worin
- R⁴ und R⁵: gemeinsam mit dem Stickstoffatom, an das sie gebunden sind, einen Morpholinyl- oder Thiomorpholinyl-Ring bilden,
und deren Salze.

Besonders bevorzugt sind erfindungsgemäße Verbindungen der Formeln (I) bzw. (I a), worin
- R¹, R²: unabhängig voneinander Fluor, Chlor oder Brom bedeuten,
und deren Salze.

Ganz besonders bevorzugt sind erfindungsgemäße Verbindungen der Formeln (I) bzw. (I a), worin
- R¹, R²: unabhängig voneinander Fluor, Chlor oder Brom bedeuten und
- R⁴ und R⁵: gemeinsam mit dem Stickstoffatom, an das sie gebunden sind, einen Morpholinyl- oder Thiomorpholinyl-Ring bilden,
und deren Salze.

Alkyl steht im Rahmen der Erfindung für einen linearen oder verzweigten Alkylrest mit 1 bis 4 Kohlenstoffatomen, wie z.B. Methyl, Ethyl, Propyl, Isopropyl und tert.-Butyl.

Alkoxycarbonyl steht im Rahmen der Erfindung für einen linearen oder verzweigten Alkoxycarbonylrest mit 1 bis 4 Kohlenstoffatomen, wie z.B. Methoxycarbonyl, Ethoxycarbonyl und Propoxycarbonyl.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren und deren jeweilige Mischungen. Die Racemformen lassen sich ebenso wie die Diastereomeren in an sich bekannter Weise in die stereoisomer einheitlichen Bestandteile trennen.

Die erfindungsgemäßen Verbindungen schließen die Isomeren der Formeln (I) und (Ia) sowie deren Mischungen ein. Die erfindungsgemäßen Verbindungen können auch als Salze vorliegen. Im Rahmen der Erfindung sind physiologisch unbedenkliche Salze bevorzugt.

Physiologisch unbedenkliche Salze können Salze anorganischer oder organischer Säuren sein. Bevorzugt werden Salze anorganischer Säuren wie beispielsweise Salzsäure, Bromwasserstoffsäure, Phosphorsäure oder Schwefelsäure, oder Salze organischer Carbon- oder Sulfonsäuren wie beispielsweise Essigsäure, Maleinsäure, Fumarsäure, Äpfelsäure, Zitronensäure, Weinsäure, Milchsäure, Benzoesäure oder Methansulfonsäure, Ethansulfonsäure, Phenylsulfonsäure, Toluolsulfonsäure oder Naphthalindisulfonsäure.

Physiologisch unbedenkliche Salze können auch Metall- oder Ammoniumsalze der erfindungsgemäßen Verbindungen sein. Besonders bevorzugt sind z. B. Natrium-, Kalium-, Magnesium- oder Calciumsalze sowie Ammoniumsalze, die von Ammoniak oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin abgeleitet sind.

Die erfindungsgemäßen Verbindungen (I) können hergestellt werden, indem man
[A] Aldehyde der Formel 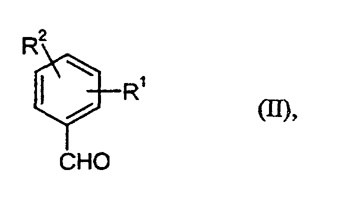 worin R¹ und R² die oben angegebenen Bedeutungen besitzen,
   zunächst mit β-Ketoestern der Formel 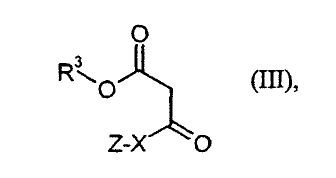 worin R³, X und Z die oben angegebenen Bedeutungen besitzen,
   mit oder ohne Basen- bzw. Säurezusatz gegebenenfalls in Gegenwart inerter organischer Lösemittel in Benzyliden-Verbindungen der Formel 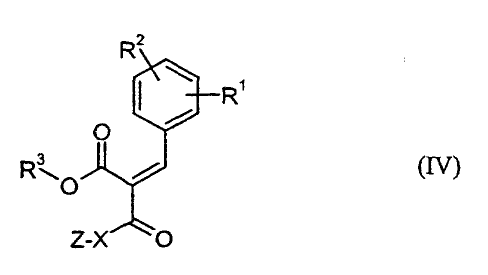 überführt und diese dann mit Amidinen der Formel 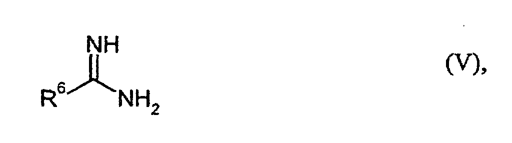 worin
   - R⁶: die oben angegebene Bedeutung hat,
   oder deren Salzen (wie z.B. Hydrochloriden oder Acetaten) mit oder ohne Basen- bzw. Säurezusatz gegebenenfalls in Gegenwart inerter organischer Lösemittel umsetzt oder
[B] Verbindungen der Formel (III) in einem einstufigen Verfahren mit Aldehyden (II) und Amidinen (V) oder deren Salzen (wie z.B. Hydrochloriden oder Acetaten) mit oder ohne Basen- bzw. Säurezusatz gegebenenfalls in Gegenwart inerter organischer Lösemittel umsetzt oder auch
[C] sofern X in Formel (I) für eine Methylengruppe steht, Verbindungen der Formel 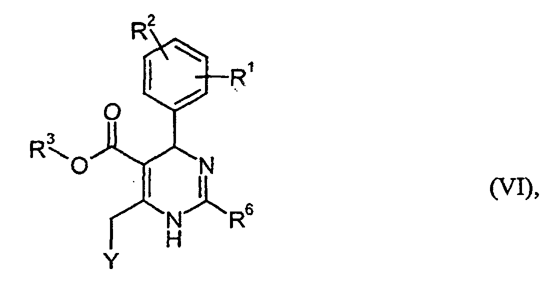
worin
- R¹, R², R³ und R⁶: die oben angegebenen Bedeutungen besitzen und
- Y: für eine nukleophil austauschbare Gruppe wie Chlorid, Bromid, Iodid, Mesylat oder Tosylat steht,
mit Verbindungen der Formel 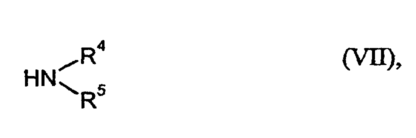 worin
- R⁴ und R⁵: die oben angegebenen Bedeutungen besitzen,
mit oder ohne Zusatz einer Hilfsbase gegebenenfalls in inerten Lösemitteln umsetzt.

Die Verbindungen (VI) können beispielsweise dadurch hergestellt werden, dass man Verbindungen der Formel 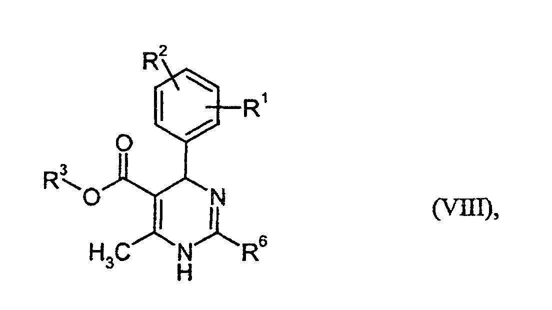 worin
- R¹, R², R³ und R⁶: die oben angegebenen Bedeutungen besitzen,
mit einem Bromierungsmittel, wie z. B. N-Bromsuccinimid, vorzugsweise in Gegenwart inerter Lösemittel, in Verbindungen der Formel 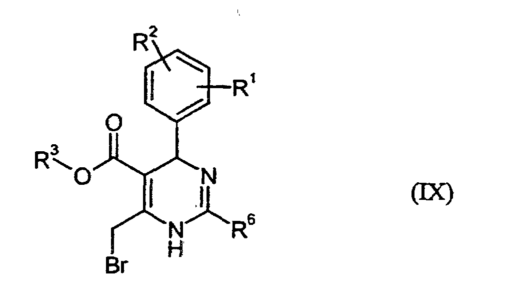 überführt.

Diese können dann direkt oder nach weiterer, literaturüblicher Transformation der nukleophil austauschbaren Gruppe mit Verbindungen (VII) umgesetzt werden.
[D] Sofern X in Formel (I) für eine Ethylengruppe steht, kann man auch Verbindungen der Formel 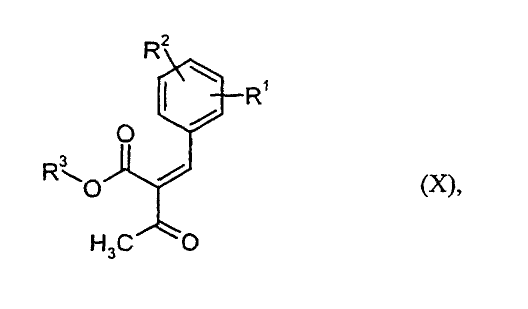
worin
- R¹, R² und R³: die oben angegebenen Bedeutungen besitzen,
mit Immoniumsalzen der Formel 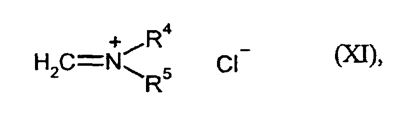 worin
- R⁴ und R⁵: die oben angegebenen Bedeutungen besitzen,
mit oder ohne Zusatz einer Hilfsbase gegebenenfalls in inerten Lösemitteln in Verbindungen der Formel 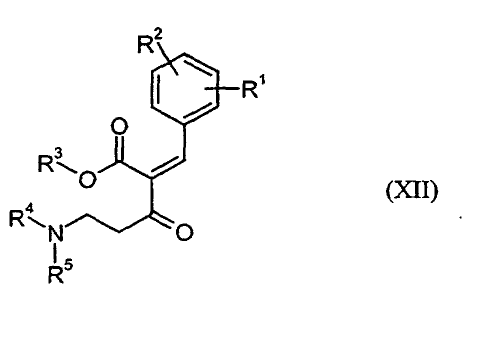 überführen und diese dann mit Amidinen (V) oder deren Salzen (wie z.B. Hydrochloriden oder Acetaten) mit oder ohne Basen- bzw. Säurezusatz gegebenenfalls in Gegenwart inerter organischer Lösemittel umsetzen.

Für die Herstellung der erfindungsgemäßen Verbindungen der Formel (I), worin X für eine Methylengruppe und Z für die Gruppe -NR⁴R⁵ stehen, können die entsprechenden ß-Ketocarbonsäureester (III) auch durch Umsetzung von Chloracetessigestem der Formel 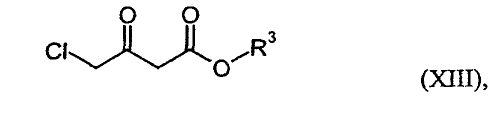 worin
- R³: die oben angegebene Bedeutung hat,
mit Verbindungen der Formel (VII) erhalten werden.

Für die Herstellung der erfindungsgemäßen Verbindungen der Formel (I), worin X für eine Ethylengruppe und Z für Pyridyl stehen, können die entsprechenden β-Ketocarbonsäureester (III) auch durch Umsetzung der Dianionen von Acetessigestem der Formel 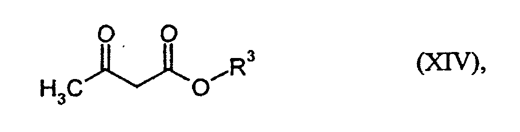 worin
- R³: die oben angegebene Bedeutung hat,
mit Picolyl-Derivaten der Formel 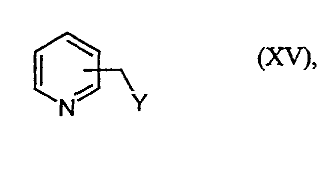 worin.
- Y: die oben angegebene Bedeutung hat,
erhalten werden.

Die als Ausgangsstoffe verwendeten Aldehyde (II) sind bekannt oder können nach literaturbekannten Methoden herstellt werden [vgl. T.D. Harris und G.P. Roth, J. Org. Chem. 44, 146 (1979); DE-OS 2 165 260 und 2 401 665,; Mijano et al., Chem. Abstr. 59, 13 929 c (1963); E. Adler und H.-D. Becker, Chem. Scand. 15, 849 (1961); E.P. Papadopoulos, M. Mardin und Ch. Issidoridis, J. Org. Chem. Soc. 78, 2543 (1956)].

Die als Ausgangsstoffe verwendeten β-Ketocarbonsäureester (III) sind teilweise bekannt oder können analog literaturbekannten Methoden hergestellt werden [z.B. D. Borrmann, "Umsetzung von Diketen mit Alkoholen, Phenolen und Mercaptanen", in "Methoden der Organischen Chemie" (Houben-Weyl), Bd. VII/4, 230 ff (1968); Y. Oikawa, K. Sugano und O. Yonemitsu, J. Org. Chem. 43, 2087 (1978)].

Die Verbindungen (V) sind teilweise bekannt oder können wie in WO-A-99/54326 und WO-A-99/54329 beschrieben hergestellt werden.

Die Verbindungen (VTII) und (X) können entsprechend der Verfahrensvarianten [A] oder [B], wie in WO-A-99/54326 beschrieben, hergestellt werden.

Die Verbindungen (VII) und (XI) sind bekannt oder nach üblichen Methoden herstellbar.

Für alle Verfahrensvarianten A, B, C, D und E kommen als Lösemittel alle inerten organischen Lösemittel in Frage. Hierzu gehören vorzugsweise Alkohole wie Methanol, Ethanol, Isopropanol, Ether wie Dioxan, Diethylether, Tetrahydrofuran, Glykolmonomethylether, Glykoldimethylether, Carbonsäuren wie Eisessig, oder Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150°C, vorzugsweise jedoch bei der Siedetemperatur des jeweiligen Lösemittels.

Die Umsetzung kann bei Normaldruck, aber auch bei erhöhtem Druck durchgeführt werden. Im allgemeinen arbeitet man unter Normaldruck.

Die Umsetzung kann mit oder ohne Basen- bzw. Säurezusatz durchgeführt werden; es empfiehlt sich jedoch, die Umsetzung in Gegenwart von schwächeren Säuren, wie z.B. Essigsäure oder Ameisensäure, durchzuführen.

Verbindungen der Formel (IX) sind neu; die Erfindung betrifft deshalb auch Verbindungen der Formel (IX).

Eine Ausführungsform der Erfindung betrifft Kombinationen von A) mindestens eines der oben definierten Dihydropyrimidine, B) mindestens eines von A verschiedenen anderen antiviralen Mittels.

Eine besondere Ausfühningsform der Erfindung betrifft Kombinationen A) obigen Dihydropyrimidinen, B) HBV-Polymerase-Inhibitoren und gegebenenfalls C) Immunmodulatoren.

Bevorzugte Immunmodulatoren C) umfassen beispielsweise sämtliche Interferone wie α-, β- und γ-Interferone, insbesondere auch α-2a- und α-2b-Interferone, Interleukine wie Interleukin-2, Polypeptide wie Thymosin-α-1 und Thymoctonan, Imidazochinolinderivate wie ® Levamisole, Immunglobuline und therapeutische Vaccine.

Die Erfindung betrifft also auch diese Kombinationen zur Behandlung und Prophylaxe von HBV-Infektionen sowie ihre Verwendung zur Behandlung HBV-induzierter Erkrankungen.

Die Verwendung der erfindungsgemäßen Kombinationen bietet bei der Behandlung HBV-induzierter Erkrankungen wertvolle Vorteile im Vergleich zur Monotherapie mit den Einzelverbindungen, nämlich hauptsächlich eine synergistische antivirale Wirksamkeit, aber auch eine gute Verträglichkeit der erfindungsgemäßen Kombinationen im Bereich der Toxizität, bei der 50 % der Zellen überleben ("Tox-50") - im Vergleich zur Tox-50 der Einzelkomponenten.

Als HBV-Polymerase-Inhibitoren B im Sinne der Erfindung werden solche Stoffe bezeichnet, die im nachfolgend beschriebenen endogenen Polymerase-Assay, das von Ph. A. Furman et al. in Antimicrobial Agents and Chemotherapy, Vol. 36 (No. 12), 2688 (1992) publiziert worden ist, zu einer Hemmung der Bildung eines HBV-DNA-Doppelstranges derart führen, dass sich maximal 50 % der Aktivität des Nullwerts ergeben:

HBV-Virionen aus Kulturüberständen bauen in vitro Nucleosid-5'-triphosphate in den Plusstrang der HBV-DNA ein. Unter Verwendung von Agarosegel-Elektrophorese wird der Einbau von [α-³²P]-Deoxynucleosid-5'-triphosphat in das virale 3.2-kb DNA-Produkt in An- und Abwesenheit einer Substanz mit potentiell HBV-Polymerase-hemmenden Eigenschaften beobachtet. HBV-Virionen werden aus dem Zellkultur-Überstand von HepG2.2.15-Zellen durch Fällung mit Polyethylenglykol gewonnen und aufkonzentriert. 1 Volumenteil geklärter Zellkulturüberstand wird mit ¼ Volumenteil einer wässrigen Lösung enthaltend 50 Gew.-% Polyethylenglykol 8000 und 0.6 M Kochsalz gemischt. Die Virionen werden durch Zentrifugieren bei 2,500 x g/15 Minuten sedimentiert. Die Sedimente werden in 2 ml Puffer enthaltend 0.05 M Tris-HCl (pH 7.5) resuspendiert und gegen den gleichen Puffer enthaltend 100 mM Kaliumchlorid dialysiert. Die Proben können bei -80°C eingefroren werden. Jeder Reaktionsansatz (100 µl) enthält mindestens 10⁵ HBV-Virionen; 50 mM Tris-HCI (pH 7.5); 300 mM Kaliumchlorid; 50 mM Magnesiumchlorid; 0.1 % ® Nonident P-40 (nichtionisches Detergens der Fa. Boehringer Mannheim); je 10 µM dATP, dGTP und dTTP; 10 µCi [³²P]dCTP (3000 Ci/mmol; Endkonzentration 33 nM) und 1 µM des potentiellen Polymerase-Inhibitors in seiner triphosphorylierten Form. Die Proben werden bei 37°C eine Stunde lang inkubiert, und dann wird die Reaktion durch Zugabe von 50 mM EDTA gestoppt. Eine 10 %-ige Gewichtsvolumen-SDS-Lösung (enthaltend 10 g SDS pro 90 ml Wasser) wird bis zu einer Endkonzentration von 1 Vol.-% (bezogen auf Gesamtvolumen) zugegeben, und Proteinase K wird bis zu einer Endkonzentration von 1 mg/ml zugegeben. Nach Inkubation bei 37°C für eine Stunde werden Proben mit demselben Volumen Phenol/Chloroform/Isoamylalkohol (Volumen-Verhältnis 25:24:1) extrahiert, und aus der wässrigen Phase wird die DNA mit Ethanol gefällt. Das DNA-Pellet wird in 10 µl Gelpuffer (Lösung von 10.8 g Tris, 5.5 g Borsäure und 0.75 g EDTA in 1 Liter Wasser (= TBE-Puffer)) resuspendiert und durch Elektrophorese in einem Agarosegel getrennt. Das Gel wird entweder getrocknet oder die darin enthaltenen Nukleinsäuren mittels Southem-Transfertechnik auf eine Membran übertragen. Danach wird die Menge des gebildeten und markierten DNA-Doppelstranges im Verhältnis zur Negativkontrolle (= Endo-Pol-Reaktion ohne Substanz oder mit Kontrollsubstanz ohne Wirkung) bestimmt. Ein HBV-Polymerase-Inhibitor liegt dann vor, wenn maximal 50% der Aktivität der Negativkontrolle vorliegen.

Bevorzugte HBV-Polymerase-Inhibitoren B) umfassen beispielsweise
3TC = Lamivudin =
= 4-Amino-1-[(2R-cis)-2-(hydroxymethyl)-1.3-oxathiolan-5-yl]-pyrimidin-2(1H)-on, vgl. EP-PS 382 526 (= US-PS 5 047 407) und WO 91/11186 (= US-PS 5 204 466);
Adefovir Dipivoxil =
9-{2-[[Bis[(Pivaloyloxy)-methoxy]-phosphinyl]-methoxy]-ethyl}-adenin, vgl. EP-PS 481 214 (= US-PS 5 663 159 und 5 792 756), US-PS 4 724 233 und 4 808 716;
BMS 200 475 =
[1S-(1.α,3.α,4.β)]-2-Amino-1.9-dihydro-9-[4-hydroxy-3-(hydroxymethyl)-2-methylen-cyclopentyl]-6H-purin-6-on, vgl. EP-PS 481 754 (= US-PS 5 206 244 und 5 340 816), WO 98/09964 und 99/41275;
Abacavir =
(-)-(1S-cis)-4-[2-Amino-6-(cyclopropylamino)-9H-purin-9-yl]-2-cyclopenten-1-methanol, vgl. EP-PS 349 242 (= US-PS 5 049 671) und EP-PS 434 450 (= US-PS 5 034 394);
FTC =
(2R-cis)-4-Ammo-5-fluor-1-[2-(hydroxymethyl)-1.3-oxathiolan-5-yl]-pyrimidin-2(1H)-on, vgl. WO 92/14743 (= US-PS 5 204 466, 5 210 085, 5 539 116, 5 700 937, 5 728 575,5 814 639, 5 827 727, 5 852 027, 5 892 025, 5 914 331,5 914 400) und WO 92/18517;
β-L-FDDC =
5-(6-Amino-2-fluor-9H-purin-9-yl)-tetrahydro-2-furanmethanol, vgl. WO 94/27616 (= US-PS 5 627 160, 5 561 120,5 631 239 und 5 830 881);
L-FMAU = 1-(2-Deoxy-2-fluor-β-L-arabinofuranosyl)-5-methyl-pyrimidin-2.4(1H, 3H)-dion, vgl. WO 99/05157, WO 99/05158 und US-PS 5 753 789.

Eine weitere bevorzugte Ausführungsform der Erfindung betrifft Kombinationen von A) obigen Dihydropyrimidinen (I) bzw. (Ia) und B) Lamivudin.

Andere bevorzugte HBV-antivirale Mittel B umfassen z.B. Phenylpropenamide der Formel 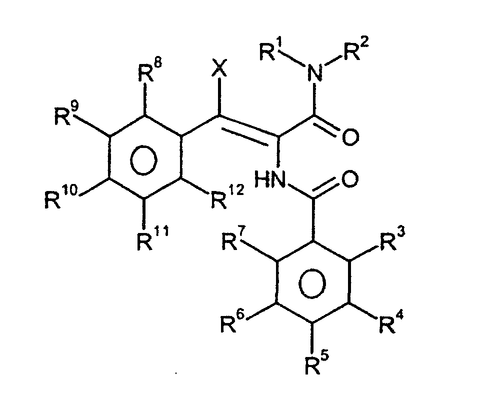 worin
- R¹ und R²: unabhängig voneinander C₁-C₄-Alkyl bedeuten oder zusammen mit dem Stickstoffatom, an dem sie stehen, einen Ring mit 5 bis 6 Ringatomen, die Kohlenstoff und/oder Sauerstoff umfassen, bilden,
- R³ bis R¹²: unabhängig voneinander Wasserstoff, Halogen, C₁-C₄-Alkyl, gegebenenfalls substituiertes C₁-C₄-Alkoxy, Nitro, Cyano oder Trifluormethyl,
- R¹³: Wasserstoff, C₁-C₄-Alkyl, C₁-C₇-Acyl oder Aralkyl und
- X: Halogen oder gegebenenfalls substituiertes C₁-C₄-Alkyl
bedeuten, und deren Salze.

Diese Phenylpropenamide und Verfahren zu ihrer Herstellung sind aus der WO 98/33501 bekannt, auf die hiermit zum Zwecke der Offenbarung Bezug genommen wird. AT-61 ist die Verbindung der obigen Formel, worin X Chlor, A 1-Piperidinyl und Y und Z jeweils Phenyl bedeuten.

Bevorzugte Immunmodulatoren C) umfassen beispielsweise sämtliche Interferone wie α-, β- und γ-Interferone, insbesondere auch α-2a- und α-2b-Interferone, Interleukine wie Interleukin-2, Polypeptide wie Thymosin-α-1 und Thymoctonan, Imidazochinolinderivate wie ® Levamisole, Immunglobuline und therapeutische Vaccine.

Eine weitere bevorzugte Ausführungsform der Erfindung betrifft Kombinationen von A) obigen Dihydropyrimidinen (I) bzw. (Ia), B) Lamivudin und gegebenbenfalls C) Interferon.

### Testbeschreibung

Die antivirale Wirkung der erfindungsgemäßen Verbindungen gegen das Hepatitis-B-Virus wurde in Anlehnung an die von M.A. Sells et al., Proc. Natl. Acad. Sci. 84, 1005-1009 (1987) und B.E. Korba et al., Antiviral Research 19, 55-70 (1992) beschriebenen Methoden untersucht.

Die antiviralen Tests wurden in 96-well-Mikrotiterplatten durchgeführt. Die erste vertikale Reihe der Platte erhielt nur Wachstumsmedium und HepG2.2.15-Zellen. Sie diente als Viruskontrolle.

Stammlösungen der Testverbindungen (50 mM) wurden zunächst in DMSO gelöst, weitere Verdünnungen wurden in Wachstumsmedium der HepG2.2.15 hergestellt. Die erfindungsgemäßen Verbindungen wurden in der Regel in einer Testkonzentration von 100 µM (1. Testkonzentration) jeweils in die zweite vertikale Testreihe der Mikrotiterplatte pipettiert und anschließend in Zweierschritten 2¹⁰-fach in Wachstumsmedium plus 2 Gew.-% fötales Kälberserum verdünnt (Volumen 25 µl).

Jeder Napf der Mikrotiterplatte erhielt dann 225 µl einer HepG2.2.15-Zellsuspension (5 x 10⁴ Zellen/ml) in Wachstumsmedium plus 2 Gew.-% fötales Kälberserum. Der Testansatz wurde 4 Tage bei 37°C und 5 % CO₂ (v/v) inkubiert.

Anschließend wurde der Überstand abgesaugt und verworfen, und die Näpfe erhielten 225 µl frisch zubereitetes Wachstumsmedium. Die erfindungsgemäßen Verbindungen wurden jeweils erneut als 10-fach konzentrierte Lösung in einem Volumen von 25 µl zugefügt. Die Ansätze wurden weitere 4 Tage inkubiert.

Vor der Ernte der Überstände zur Bestimmung des antiviralen Effektes wurden die HepG2.2.15-Zellen lichtmikroskopisch oder mittels biochemischer Nachweisverfahren (z.B. Alamar-Blue-Färbung oder Trypanblau-Färbung) auf zytotoxische Veränderungen untersucht.

Anschließend wurden die Überstände und/oder Zellen geerntet und mittels Vakuum auf mit Nylonmembran bespannten 96-Napf-Dot-Blot-Kammern (entsprechend den Herstellerangaben) gesogen.

### Zytotoxizitätsbestimmung

Substanzinduzierte zytotoxische oder zytostatische Veränderungen der HepG2.2.15-Zellen wurden z.B. lichtmikroskopisch als Änderungen der Zellmorphologie ermittelt. Derartige Substanz-induzierte Veränderungen der HepG2.2.15-Zellen im Vergleich zu unbehandelten Zellen wurden z.B. als Zellyse, Vakuolisierung oder veränderte Zellmorphologie sichtbar. 50 % Zytotoxizität (Tox.-50) bedeuten, dass 50% der Zellen eine der entsprechenden Zellkontrolle vergleichbare Morphologie aufweisen.

Die Verträglichkeit einiger der erfindungsgemäßen Verbindungen wurde zusätzlich auf anderen Wirtszellen wie z.B. HeLa-Zellen, primäre periphere Blutzellen des Menschen oder transformierte Zellinien wie H-9-Zellen, getestet.

Es konnten keine zytotoxischen Veränderungen bei Konzentrationen der erfindungsgemäßen Verbindungen von >10 µM festgestellt werden.

### Bestimmung der antiviralen Wirkung

Nach Transfer der Überstände oder lysierten Zellen auf die Nylon-Membran der Blot-Apparatur (s.o.) wurden die intra- oder extrazellulären Überstände der HepG2.2.15-Zellen denaturiert (1.5 M NaCl/0.5 N NaOH), neutralisiert (3 M NaCl/0.5 M Tris HCI, pH 7.5) und gewaschen (2 x SSC). Anschließend wurde die DNA durch Inkubation der Filter bei 120°C 2-4 Stunden an die Membran gebacken.

### Hybridisierung der DNA

Der Nachweis der viralen DNA von den behandelten HepG2.2.15-Zellen auf den Nylonfiltem wurde in der Regel mit nichtradioaktiven, Digoxigenin-markierten Hepatitis-B-spezifischen DNA-Sonden durchgeführt, die jeweils nach Herstellerangabe mit Digoxigenin markiert, gereinigt und zur Hybridisierung eingesetzt wurden.

Die Prähybridisierung und Hybridisierung erfolgten in 5 x SSC, 1 x Blockierungsreagenz, 0,1 Gew.-% N-Lauroylsarcosin, 0,02 Gew.-% SDS und 100 µg Sperma-DNA des Herings. Die Prähybridisierung erfolgte 30 Minuten bei 60°C, die spezifische Hybridisierung mit 20 bis 40 ng/ml der digoxigenierten, denaturierten HBV-spezifischen DNA (14 Stunden, 60°C). Anschließend wurden die Filter gewaschen.

### Nachweis der HBV-DNA durch Digoxigenin-Antikörper

Der immunologische Nachweis der Digoxigenin-markierten DNA erfolgte nach Herstellerangaben:

Die Filter wurden gewaschen und in einem Blockierungsreagenz (nach Herstellerangabe) prähybridisiert. Anschließend wurde mit einem Anti-DIG-Antikörper, der mit alkalischer Phosphatase gekoppelt war, 30 Minuten hybridisiert. Nach einem Waschschritt wurde das Substrat der alkalischen Phosphatase, CSPD, zugefügt, 5 Minuten mit den Filtern inkubiert, anschließend in Plastikfolie eingepackt und weitere 15 Minuten bei 37°C inkubiert. Die Chemilumineszenz der Hepatitis-B-spezifischen DNA-Signale wurde über eine Exposition der Filter auf einem Röntgenfilm sichtbar gemacht (Inkubation je nach Signalstärke: 10 Minuten bis 2 Stunden).

Die halbmaximale Hemmkonzentration (IC₅₀, inhibitorische Konzentration 50 %) wurde als die Konzentration bestimmt, bei der gegenüber einer unbehandelten Probe die intra- oder extrazelluläre Hepatitis-B-spezifische Bande durch die erfindungsgemäße Verbindung um 50 % reduziert wurde.

Die erfindungsgemäßen Verbindungen zeigen eine nicht vorhersehbare wertvolle Wirkung gegen Viren. Sie sind überraschenderweise antiviral gegen Hepatitis-B-Viren (HBV) wirksam, indem sie eine außerordentlich starke Reduktion intra- und/oder extrazellulärer HBV-DNA verursachen. Die erfindungsgemäßen Verbindungen sind somit zur Behandlung von virusinduzierten Erkrankungen, insbesondere von akut und chronisch persistenten Virusinfektionen des HBV geeignet. Eine chronische Viruserkrankung, hervorgerufen durch das HBV, kann zu unterschiedlich schweren Krankheitsbildern führen; bekanntermaßen führt die chronische Hepatitis-B-Virusinfektion in vielen Fällen zur Leberzirrhose und/oder zum hepatozellulären Karzinom.

Als Indikationsgebiete für die erfindungsgemäßen Verbindungen können beispielsweise genannt werden:

die Behandlung von akuten und chronischen Virusinfektionen, die zu einer infektiösen Hepatitis führen können, beispielsweise die Infektionen mit Hepatitis-B-Viren. Besonders geeignet sind die erfindungsgemäßen Verbindungen zur Behandlung von chronischen Hepatitis-B-Infektionen und die Behandlung von akuten und chronischen Hepatitis-B-Virusinfektionen.

Zur vorliegenden Erfindung gehören pharmazeutische Zubereitungen, die neben nichttoxischen, inerten pharmazeutisch geeigneten Trägerstoffen eine oder mehrere Verbindungen (I) bzw. (Ia) bzw. eine erfindungsgemäße Kombination. enthalten oder die aus einem oder mehreren Wirkstoffen (I) bzw. (Ia) bzw. aus einer erfindungsgemäßen Kombination bestehen.

Die Wirkstoffe (I) bzw. (Ia) sollen in den oben aufgeführten pharmazeutischen Zubereitungen in einer Konzentration von etwa 0,1 bis 99,5 Gew.-%, vorzugsweise von etwa 0,5 bis 95 Gew.-% der Gesamtmischung, vorhanden sein.

Die oben aufgeführten pharmazeutischen Zubereitungen können außer den Verbindungen (I) bzw. (Ia) auch weitere pharmazeutische Wirkstoffe enthalten.

Das Mengenverhältnis der Komponenten A, B und gegebenenfalls C der erfindungsgemäßen Kombinationen kann innerhalb weiter Grenzen schwanken; vorzugsweise beträgt es 5 bis 500 mg A / 10 bis 1000 mg B, insbesondere 10 bis 200 mg A / 20 bis 400 mg B.

Die gegebenenfalls mitzuverwendende Komponente C kann in Mengen von vorzugsweise 1 bis 10 Millionen, insbesondere 2 bis 7 Millionen I.E. (internationale Einheiten), etwa dreimal wöchentlich über einen Zeitraum bis zu einem Jahr angewandt werden.

Die erfindungsgemäßen Verbindungen oder Kombinationen sollen in den oben aufgeführten pharmazeutischen Zubereitungen im allgemeinen in einer Konzentration von etwa 0,1 bis 99,5, vorzugsweise etwa 0,5 bis 95, Gew.-% der Gesamtmischung vorhanden sein.

Die Herstellung der oben aufgeführten pharmazeutischen Zubereitungen kann auf übliche Weise nach bekannten Methoden, z.B. durch Mischen des oder der Wirkstoffe mit dem oder den Trägerstoffen, erfolgen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise von 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels, sowie dem Zeitraum bzw. Intervall, innerhalb welchem die Verabreichung erfolgt.

Weiterer Gegenstand der Erfindung sind daher die oben definierten Verbindungen und Kombinationen zur Bekämpfung von Erkrankungen.

Weiterer Gegenstand der Erfindung sind Arzneimittel, enthaltend mindestens eine der oben definierten Verbindungen oder Kombinationen und gegebenenfalls einen oder mehrere weitere pharmazeutische(n) Wirkstoff(e).

Weiterer Gegenstand der Erfindung ist die Verwendung der oben definierten Verbindungen und Kombinationen bei der Herstellung eines Arzneimittels zur Behandlung und Prophylaxe der oben beschriebenen Erkrankungen, vorzugsweise von Viruserkrankungen, insbesondere von Hepatitis B.

Die Prozentangaben der nachfolgenden Beispiele beziehen sich, sofern nicht anders angegeben, jeweils auf das Gewicht. Die Verhältnisse von Lösemitteln in Lösemittelgemischen beziehen sich jeweils auf das Volumen.

### Beispiele

### A. Ausgangsverbindungen

### Beispiel I:3-Fluorpyridin-N-oxid

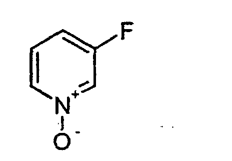

Zu einer Lösung von 11,10 g (114,324 mmol) 3-Fluorpyridin in 74,00 ml Essigsäure gibt man 22,20 ml H₂O₂ (30%ig) und läßt 7 Stunden bei 100°C Badtemperatur rühren. Danach wird bis auf 30 ml eingeengt, 30 ml Wasser zugefügt und wieder auf 30 ml eingeengt. Die Lösung wird mit Dichlormethan verrührt, durch Zugabe von K₂CO₃ basisch gestellt, getrennt, die wässrige Phase zweimal mit Dichlormethan ausgeschüttelt, getrocknet und eingeengt.
Ausbeute: 11,5 g (88,9 %)
Fp.:66-68°C

### Beispiel II: 2-Cyano-3-fluorpyridin

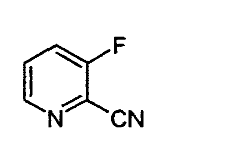

5,20 g (45,980 mmol) der Verbindung aus Beispiel I werden in 50 ml Acetonitril gelöst. Unter Argon werden 13,70 g (138,092 mmol) Trimethylsilylnitril zugegeben und langsam 12,80 ml Triethylamin zulaufen gelassen. Die Lösung wird 7 Stunden unter Rückfluß und danach über Nacht bei Raumtemperatur gerührt. Nach dem Einengen mit einer Wasserstrahlpumpe wird in Dichlormethan aufgenommen, zweimal mit 50 ml 2 N wässriger Natriumcarbonatlösung geschüttelt, mit Wasser gewaschen, getrocknet und eingeengt.
Ausbeute (roh): 5,3 g (Öl)
Säulenchromatographie: Methylenchlorid bis Methylenchlorid/Essigester (10:1)

### Beispiel III: 2-Amidino-3-fluorpyridin-Hydrochlorid

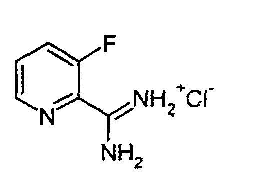

Eine Lösung von 10,30 g (84,355 mmol) der Verbindung aus Beispiel II in 30 ml Methanol wird mit einer Natriummethylat-Lösung aus 0,40 g (17,391 mmol) Natrium und 65 ml Methanol versetzt und 72 Stunden bei 20°C gerührt. 5,44 g (101,682 mmol) Ammoniumchlorid (pulverisiert) und 17,39 mmol (1,04 ml) Essigsäure werden zugegeben, 28 Stunden bei 40°C nachgerührt und abgekühlt. Es wird vom nicht löslichen Salz abgesaugt (1,78 g), eingeengt, mit Aceton eingeengt, anschließend mit Aceton verrührt, abgesaugt und gewaschen.
Ausbeute: 10,6 g
Fp.:≈ 150°C Zers.

### Beispiel IV: 2-Cyano-3,5-dichlor-pyridin

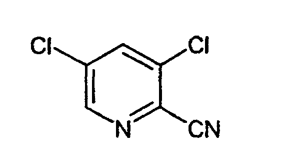

### Methode 1:

Eine Lösung von 26 g (0,158 mol) 3,5-Dichdor-pyridin-1-oxid (Johnson et al., J.Chem.Soc.B, 1967, 1211) in 80 ml Dichlormethan wird nacheinander mit 21,8 ml (0,174 mol) Trimethylsilylcyanid und 14,6 ml (0,158 mol) Dimethylcarbamidsäurechlorid versetzt und 48 Stunden bei Raumtemperatur gerührt. Es wird mit 100 ml einer 10 %-igen wässrigen NaHCO₃-Lösung versetzt und 10 Minuten intensiv gerührt. Nach Trennung der Phasen wird einmal mit Dichlormethan ausgeschüttelt; die vereinigten organischen Phasen werden getrocknet und eingeengt. Der Rückstand wird mit Dichlormethan an Kieselgel chromatographiert und aus wenig Methanol umkristallisiert.

Man erhält 11 g (40,2 %) 2-Cyano-3,5-dichlor-pyridin (Fp.: 102°C).

### Methode 2:

Analog Troschuetz, R. et al., J. Heterocycl. Chem. 33, 1815-1821 (1996) werden 150 ml Diethylenglykoldimethylether, 47,68 g (0,261 mol) 2,3,5-Trichlorpyridin, 2,0 g (0,005 mol) Tetraphenylphosphoniumbromid, 4,0 g (0,024 mol) feingepulvertes Kaliumiodid und 75,0 g (0,838 mol) Kupfer(I)cyanid unter Stickstoff zusammengegeben und 24 Stunden bei Rückfluss gerührt. Anschließend weitere 100 ml Diethylenglykoldimethylether, 2,0 g (0,005 mol) Tetraphenylphosphoniumbromid, 4,0 g (0,024 mol) feingepulvertes Kaliumiodid und 75 g (0,838 mol) Kupfer(I)cyanid hinzugegeben und man rührt weitere 89 Stunden bei Rückflußtemperatur. Nach Abkühlen auf Raumtemperatur wird abgesaugt und das Filtrat destillativ weitgehend von Diethylenglykoldimethylether befreit. Der Rückstand wird in Toluol aufgenommen und mit einer wässrigen Lösung von Mohr'schem Salz- und dann mit wässriger NaHCO₃-Lösung gewaschen (Peroxidtest). Dann wird mit Wasser frei von Diethylenglykoldimethylether gewaschen. Man filtriert über Cellit, trocknet das Filtrat über Magnesiumsulfat und engt die Lösung ein.

Man erhält 18,0 g (40,0 %) 2-Cyano-3,5-dichlorpyridin.

### Beispiel V: 2-Cyano-3,5-difluor-pyridin

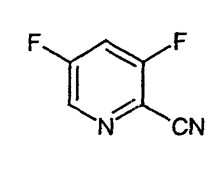

50 g (0,29 mol) 2-Cyano-3,5-dichlorpyridin aus Beispiel IV, 33,6 g (0,58 mol) Kaliumfluorid und 10 g Polyethylengykol 8000 werden mit 125 ml DMSO versetzt und 30 Minuten auf 160°C erhitzt. Nach Abkühlung wird das Produkt zusammen mit dem DMSO im Hochvakuum abdestilliert, das Destillat auf Wasser gegeben, mit Toluol extrahiert und über Natriumsulfat getrocknet. Das Produkt wird als Toluollösung weiter umgesetzt.
R_{f}-Wert: 0,43 (Cyclohexan/Essigester = 7:3)

### Beispiel VI: 3,5-Difluor-2-pyridincarboximidamid-Hydrochlorid

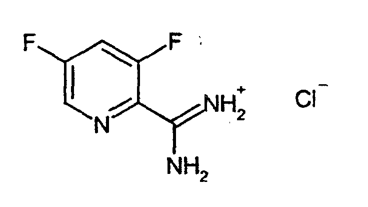

Zu einer auf 0 bis 5°C gekühlten Suspension von 33,4 g (0,624 mol) Ammoniumchlorid in 11 Toluol werden 328 ml Trimethylaluminium (2 M in Hexan, 0,624 mol) getropft; die Mischung wird bei Raumtemperatur gerührt, bis die Methanentwicklung beendet ist. Danach wird die toluolische Lösung von 2-Cyano-3,5-dichlor-pyridin aus Beispiel V zugetropft und anschließend über Nacht bei 80°C nachgerührt. Nach Kühlung auf 0 bis -5°C wird Methanol bis zum Ende der Gasentwicklung zugetropft, die Salze abgesaugt und zweimal mit wenig Methanol gewaschen. Das Lösungsmittel wird abgezogen, der Rückstand in 500 ml Dichlormethan/Methanol (9:1) gelöst und nochmals von anorganischen Salzen abgesaugt.

Nach Abziehen des Lösungsmittels verbleiben 23,6 g (39,1 %) 3,5-Difluor-2-pyridincarboximidamid-Hydrochlorid (Fp.:183°C).
¹H-NMR (DMSO-D₆):
8,3-8,45 (m, 1H) ppm; 8,8 (d, J = 2 Hz, 1H) ppm; 9,7 (s, breit, 4H) ppm.

### Beispiel VII: 2-Acetyl-3-(2-chlor-4-fluorphenyl)-acrylsäuremethylester

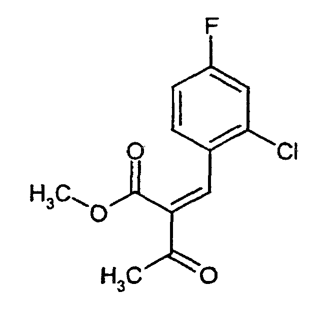

Eine Lösung von 50 g (315 mmol) 2-Chlor-fluor-benzaldehyd und 36,6 g (315 mmol) Acetessigsäuremethylester in 150 ml Isopropanol wird mit 1,7 ml Piperidinacetat versetzt. Nach Rühren über Nacht bei Raumtemperatur wird mit Dichlormethan verdünnt, mit Wasser ausgeschüttelt und die organische Phase über Natriumsulfat getrocknet und eingeengt. Das Produkt wird roh als cis/trans-Gemisch weiter umgesetzt.

### Beispiel VIII: Methyl 4-(4-morpholinyl)-3-oxobutanoat

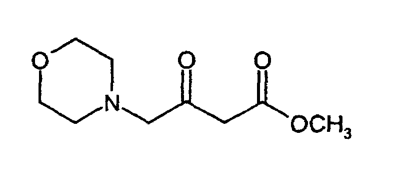

Eine Lösung von 1,0 g (6,64 mmol) 4-Chloracetessigsäuremethylester in 10 ml Dichormethan wird mit 1,27 g (14,6 mmol) Morpholin versetzt und 4. Stunden bei Raumtemperatur gerührt. Danach wird Wasser zugegeben und mit 2 N Salzsäure neutral gestellt. Die organische Phase wird über Natriumsulfat getrocknet, eingeengt, und der Rückstand wird an Kieselgel mit Cyclohexan/Ethylacetat (10:1→4:1→1:1) als Laufmittel chromatographiert.
Ausbeute: 0,475 g (35,5 %)

### Beispiel IX: 3-(2-Chlor-4-fluorphenyl)-2-[3-(1,3-thiazolidin-3-yl)-propanoyl]-acrylsäuremethylester

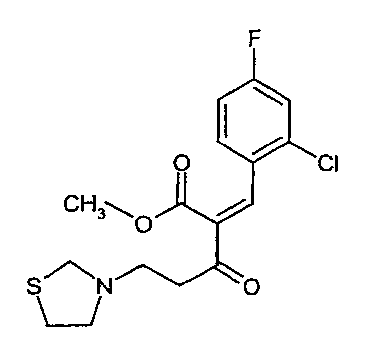

3,24 g (12,6 mmol) 2-Acetyl-3-(2-chlor-4-fluorphenyl)-acrylsäuremethylester aus Beispiel VII werden mit 1,74g (12,6 mmol) 3-Methylen-1,3-thiazolidin-3-iumchlorid [Herstellung analog H. Mohrle et al., Z. Naturforsch. 42, 1035-1046 (1987)] in 50 ml Acetonitril 48 Stunden bei 40°C gerührt. Nach Einengen wird der Rückstand in Ethylacetat aufgenommen und mit Wasser gewaschen. Die organische Phase wird über Natriumsulfat getrocknet, eingeengt und der Rückstand wird an Kieselgel mit Cyclohexan/Ethylacetat (5:1→2:1) als Laufmittel chromatographiert.
Ausbeute: 0,67 g (14, 8 %)

### Beispiel X: Methyl 3-oxo-5-(4-pyridinyl)-pentanoat

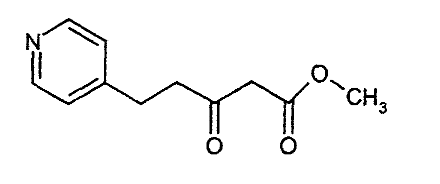

3,62 g (90,4 mmol) Natriumhydrid (als 60 %ige Suspension in Mineralöl) in 150 ml THF werden bei 0°C tropfenweise mit 10,0 g (86,1 mmol) Acetessigsäuremethylester versetzt. Nach weiterem 20-minütigem Rühren bei 0°C werden 53 ml einer 1,6 molaren Lösung von n-Butyllithium in Hexan zugetropft, 10 Minuten nachgerührt und dann eine Lösung von 14,1 g (86,1 mmol) 4-Picolylchlorid in 65 ml THF zugegeben. Nach Rühren über Nacht bei Raumtemperatur wird mit 2 N Salzsäure neutralisiert, die Phasen getrennt, die organische Phase über Natriumsulfat getrocknet, eingeengt, und das Rohprodukt durch Säulenchromatographie an Kieselgel mit zunächst Petrolether/Ethylacetat (4:1), dann mit Dichlormethan/Methanol (9:1) als Laufmittel gereinigt.
Ausbeute: 3,2 g (17,9% d.Th.)
R_{f}-Wert = 0,62 (Dichlormethan/Methanol 10:1)

### Beispiel XI: Methyl 3-(2-chlor-4-fluorphenyl)-2-[3-(4-pyridinyl)-propanoyl]-2-propenoat

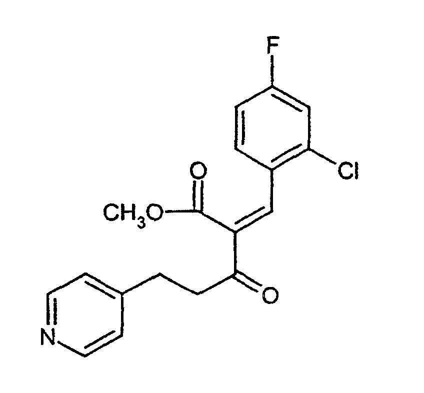

Eine Lösung von 2,44 g (15,4 mmol) 2-Chlor-4-fluor-benzaldehyd und 3,19 g (15,4 mmol) der Verbindung aus Beispiel X in 20 ml Isopropanol werden mit 0,1 ml Piperidin und 0,13 ml Eisessig versetzt. Nach Rühren über Nacht bei Raumtemperatur wird eingeengt und der Rückstand über Kieselgel mit Dichlormethan ->Dichlormethan/Methanol (50:1) als Laufmittel chromatographiert. Das Produkt wird als cis/trans-Gemisch weiter eingesetzt.
Ausbeute: 4,5 g (84,0% d.Th.)

### Beispiel XII: 4-(2-Chlor-4-fluorphenyl)-2-(3,5-difluor-2-pyridinyl)-6-methyl-1,4-dihydro-pyrimidin-5-carbonsäuremethylester

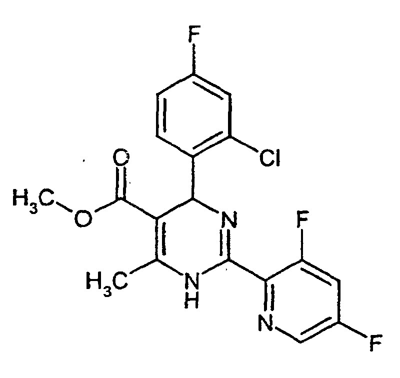

4,5 g (23,2 mmol) 3,5-Difluor-2-pyridincarboximidamid-Hydrochlorid aus Beispiel VI werden mit 7,7 g (30 mmol) 2-Acetyl-3-(2-chlor-4-fluorphenyl)-2-propen-säuremethylester aus Beispiel VII und 2,3 g (27,9 mmol) Natriumacetat in 120 ml Isopropanol gelöst bzw. suspendiert und 4 Stunden unter Rückfluß gekocht. Nach Abkühlung auf Raumtemperatur wird von anorganischen Salzen abgesaugt und eingeengt. Der Rückstand wird in einer Mischung aus 30 ml 1 N Salzsäure und 35 ml Essigester aufgenommen, und die Phasen werden getrennt. Die Essigester-Phase wird einmal mit 30 ml 1 N Salzsäure nachextrahiert. Die vereinigten wässrigen Phasen werden dreimal mit je 10 ml Diethylether ausgeschüttelt. Die wässrige Phase wird mit NaOH alkalisch gestellt und mit Essigester ausgeschüttelt. Die organischen Phasen werden über Natriumsulfat getrocknet und eingeengt.
Man erhält 7,4 g (80 %) Produkt
Fp.: 126 °C
¹H-NMR (DMSO-D₆): 2,4 (s, 3H) ppm, 3,5 (s, 3H) ppm, 6,0 (s, 1H) ppm, 7,2 (m, 1H) ppm, 7,4 (m, 2H) ppm, 8,0 (m, 1H) ppm, 8,55 (d, J = 2 Hz, 1H) ppm, 9,75 (s, NH) ppm.

Nach Trennung der Enantiomeren an chiralen Säulen (Chiralpak AS von Baker, Laufmittel n-Heptan/Ethanol = 8:2) wird das (-)-Enantiomer erhalten.
Fp.: 117 °C (aus Ethanol)
[α]_{D}²⁰: - 62.8 ° (Methanol)

### Beispiel XIII: (R)-6-Brommethyl-4-(2-chlor-4-fluorphenyl)-2-(3,5-difluor-2-pyridyl)-1,4-dihydropyrimidin-5-carbonsäurenwthylester

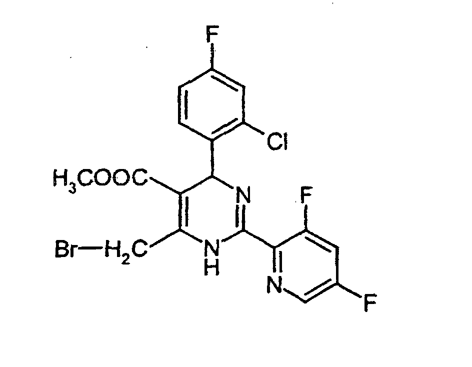

Eine Lösung von 2 g (5,05 mmol) 4-(2-Chlor-4-fluorphenyl)-2-(3,5-difluor-pyridin-2-yl)-6-methyl-1,4-dihydropyrimidin-5-carbonsäuremethylester aus Beispiel XII in 30 ml Tetrachlormethan wird unter Argon auf 50°C erhitzt, wobei eine klare Lösung entsteht. Bei dieser Temperatur werden 0,99 g (5,56 mmol) N-Bromsuccinimid zugesetzt, und die Mischung wird 10 Minuten bei dieser Temperatur gehalten. Es wird sofort abgekühlt, abgesaugt und bei Raumtemperatur unter vermindertem Druck eingeengt. Das Produkt ist laut HPLC mehr als 90%ig und wird als Rohmaterial weiter eingesetzt.
R_{f} = 0,33 (Cyclohexan/Ethylacetat = 7:3)

Analog wurden hergestellt:

### Beispiel XIV: 6-Brommethyl-4-(2,4-dichlorphenyl)-2-(3,5-difluor-2-pyridyl)-1,4-dihydropyrimidin-5-carbonsäuremethylester

### Beispiel XV: 6-Brommethyl-4-(2-chlorphenyl)-2-(3,5-difluor-2-pyridyl)-1,4-dihydropyrimidin-5-carbonsäuremethylester

### Beispiel XVI: 6-Brommethyl-4-(2,4-difluorphenyl)-2-(3,5-difluor-2-pyridyl)-1,4-dihydropyrimidin-5-carbonsäuremethylester

### Beispiel XVII: 6-Brommethyl-4-(2-chlor-4-fluorphenyl)-2-(2-thiazolyl)-1,4-dihydropyrimidin-5-carbonsäuremelhylester

### Beispiel XVIII: 6-Brommethyl-4-(2-brom-4-fluorphenyl)-2-(2-thiazolyl)-1,4-dihydropyrimidin-5-carbonsäuremethylester

### B. Herstellungsbeispiele

### Beispiel 1: 4-(2-Chlor-4-fluorphenyl)-2-(3,5-difluor-2-pyridyl)-6-(4-morpholinylmethyl)-1,4-dihydropyrimidin-5-carbonsäuremethylester

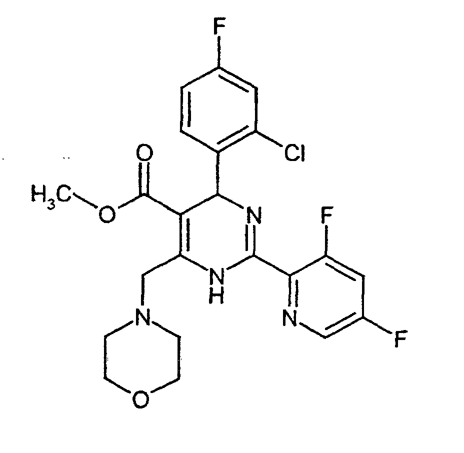

Eine Lösung von 0,38 g (2,4 mmol) 2-Chlor-4-fluorbenzaldehyd, in 10 ml Isopropanol wird zusammen mit 0,46 g (2,4 mmol) der Verbindung aus Beispiel VI, 0,48 g (2,4 mmol) der Verbindung aus Beispiel VIII und 0,24 g (2,88 mmol) Natriumacetat 2 Stunden lang unter Rückfluß erhitzt. Das Reaktionsgemisch wird eingeengt, der Rückstand in Dichlormethan aufgenommen und mit 2 N Salzsäure extrahiert. Die wässrige Phase wird mit verdünnter Ammoniak-Lösung alkalisch gestellt und mit Dichlormethan extrahiert. Die organische Phase wird mit wässriger Kochsalz-Lösung gewaschen, über Natriumsulfat getrocknet und eingeengt. Der Rückstand wird über Kieselgel mit Cyclohexan/Ethylacetat (20: 1→ 10:1) als Laufmittel chromatographiert, und das Produkt wird aus Diethylether kristallisiert.
Ausbeute: 0,03 g (2,6 %)
Fp.: 190°C

### Beispiel 2: 4-(2-Chlor-4-fluorphenyl)-2-(3,5-difluor-2-pyridyl)-6-[2-(1,3-thiazolidin-3-yl)-ethyl]-1,4-dihydropyrimidin-5-carbonsäuremethylester

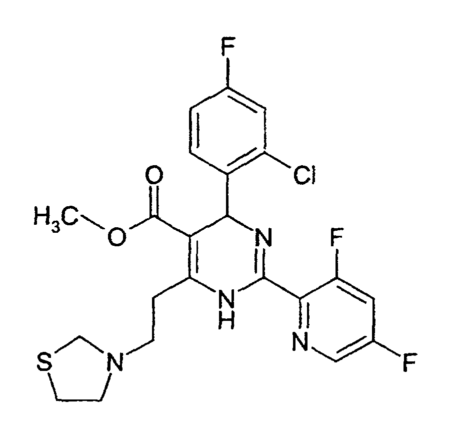

Eine Lösung von 0,20 g (0,56 mmol) der Verbindung aus Beispiel IX in 5 ml Isopropanol wird zusammen mit 0,11 g (0,56 mmol) der Verbindung aus Beispiel VI und 0,06 g (0,67 mmol) Natriumacetat 2 Stunden lang unter Rückfluß erhitzt. Das Reaktionsgemisch wird eingeengt, der Rückstand in Ethylacetat aufgenommen und mit verdünnter Salzsäure extrahiert. Die wässrige Phase wird mit verdünnter Natronlauge alkalisch gestellt und mit Ethylacetat extrahiert. Die organische Phase wird über Natriumsulfat getrocknet und eingeengt. Der Rückstand wird über Kieselgel mit Cyclohexan/Ethylacetat (5: → 3:1) als Laufmittel chromatographiert.
Ausbeute: 0,028 g (10,1 %)
Fp.: 130°C

### Beispiel 3: 4-(2-Chlor-4-fluorphenyl)-2-(3-fluor-2-pyridinyl)-6-[2-(4-pyridinyl)-ethyl]-1,4-dihydropyrimidin-5-carbonsäuremethylester

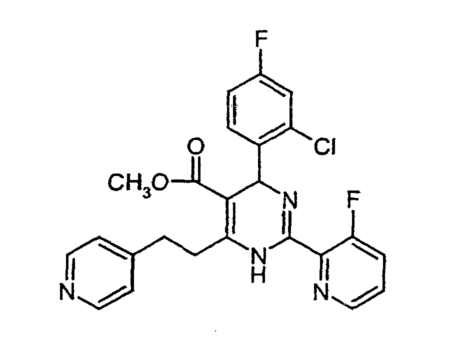

0,60 g (1,73 mmol) der Benzylidenverbindung aus Beispiel XI und 0,30g (1,73 mmol) der Verbindung aus Beispiel III werden zusammen mit 0,17 g (2,07 mmol) Natriumacetat in 12 ml Isopropanol über Nacht unter Rückfluß erhitzt. Der Ansatz wird eingeengt, in Dichlormethan aufgenommen und mit 2 N Salzsäure extrahie. Die ,wässrige Phase wird mit Natronlauge alkalisch gestellt, mit Dichlormethan extrahiert, die organische Phase mit wässriger Kochsalz-Lösung gewaschen, über Natriumsulfat getrocknet und eingeengt. Der Rückstand wird über Kieselgel mit Cyclohexan/Ethylacetat (1:1) als Laufmittel chromatographiert und durch Kristallisation aus Diethylether weiter gereinigt.
Ausbeute: 0,04 g (5% d.Th.)
R_{f}-Wert = 0,79 (Dichlormethan/Methanol 10:1)

### Beispiel 4: (R)-4-(2-Chlor-4-fluorphenyl)-2-(3,5-difluor-2-pyridyl)-6-piperidinomethyl-1,4-dihydropyrimidin-5-carbonsäuremethylester

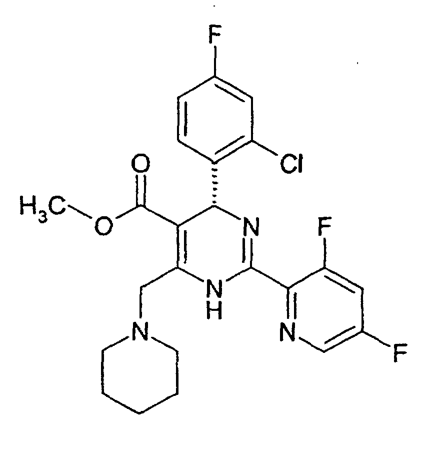

Eine Lösung von 100 mg frisch hergestelltem (R)-6-Brommethyl-4-(2-chlor-4-fluorphenyl)-2-(3,5-difluor-2-pyridyl)-1,4-dihydropyrimidin-5-carbonsäuremethylester aus Beispiel XIII in 0,5 ml Methanol wird mit 5 Äquivalenten Piperidin versetzt und bei Raumtemperatur 30 Minuten gerührt. Die Lösung wird mit Wasser verdünnt und mit Essigester extrahiert.
Ausbeute: 78 mg
Fp.: 132 °C

Die in der folgenden Tabelle aufgeführten Verbindungen wurden in analoger Weise hergestellt.

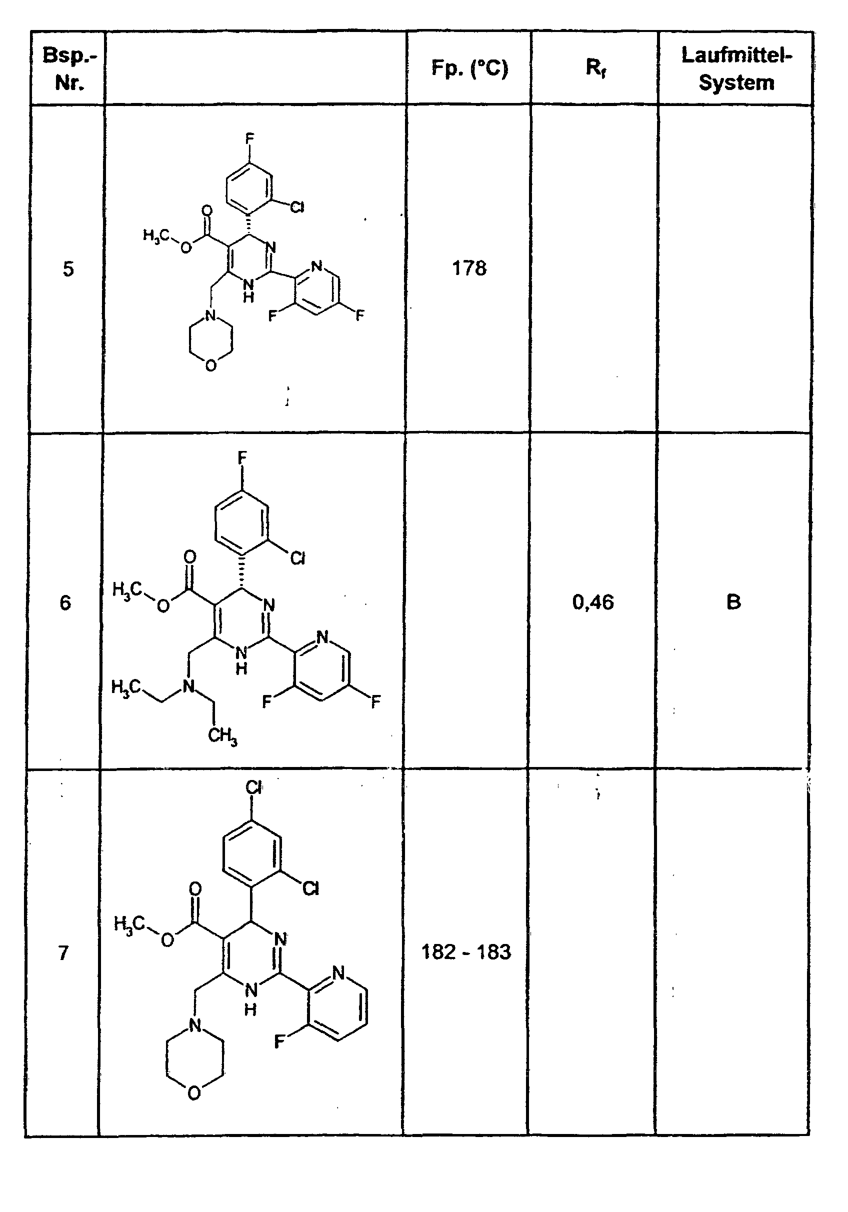

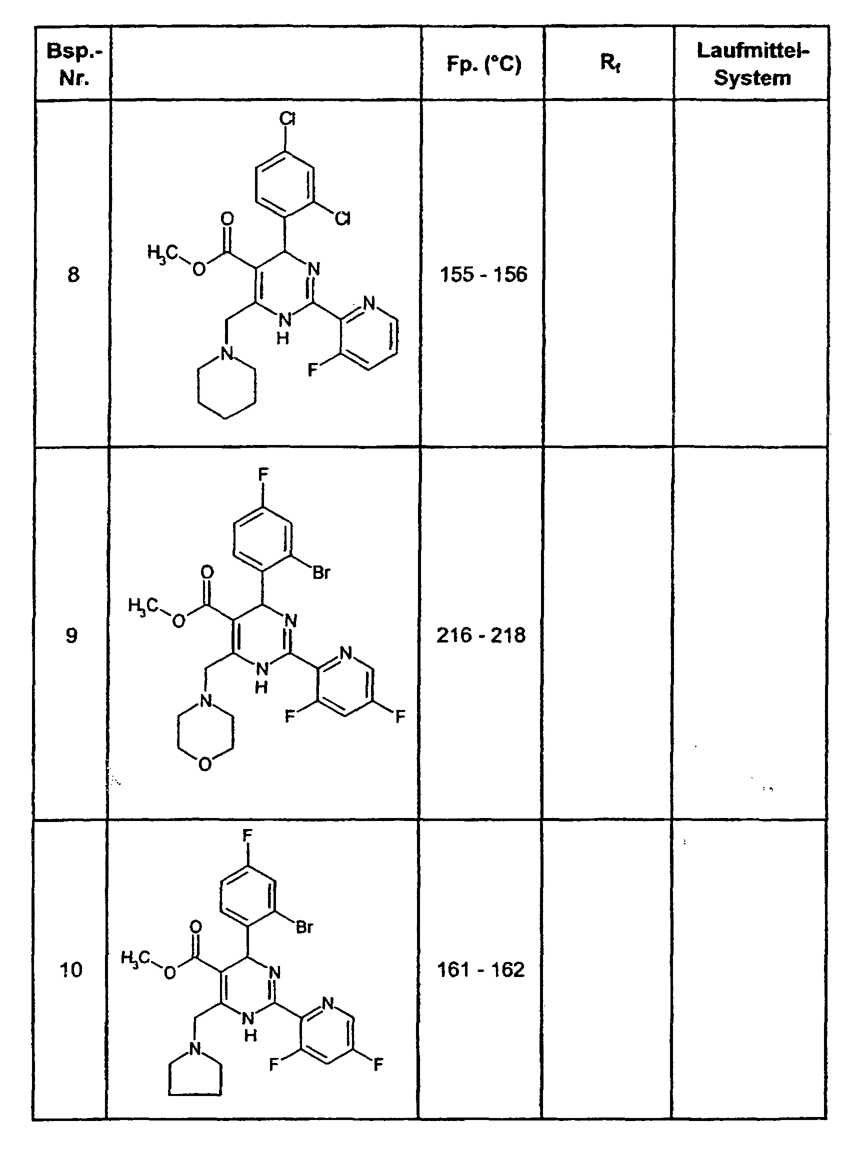

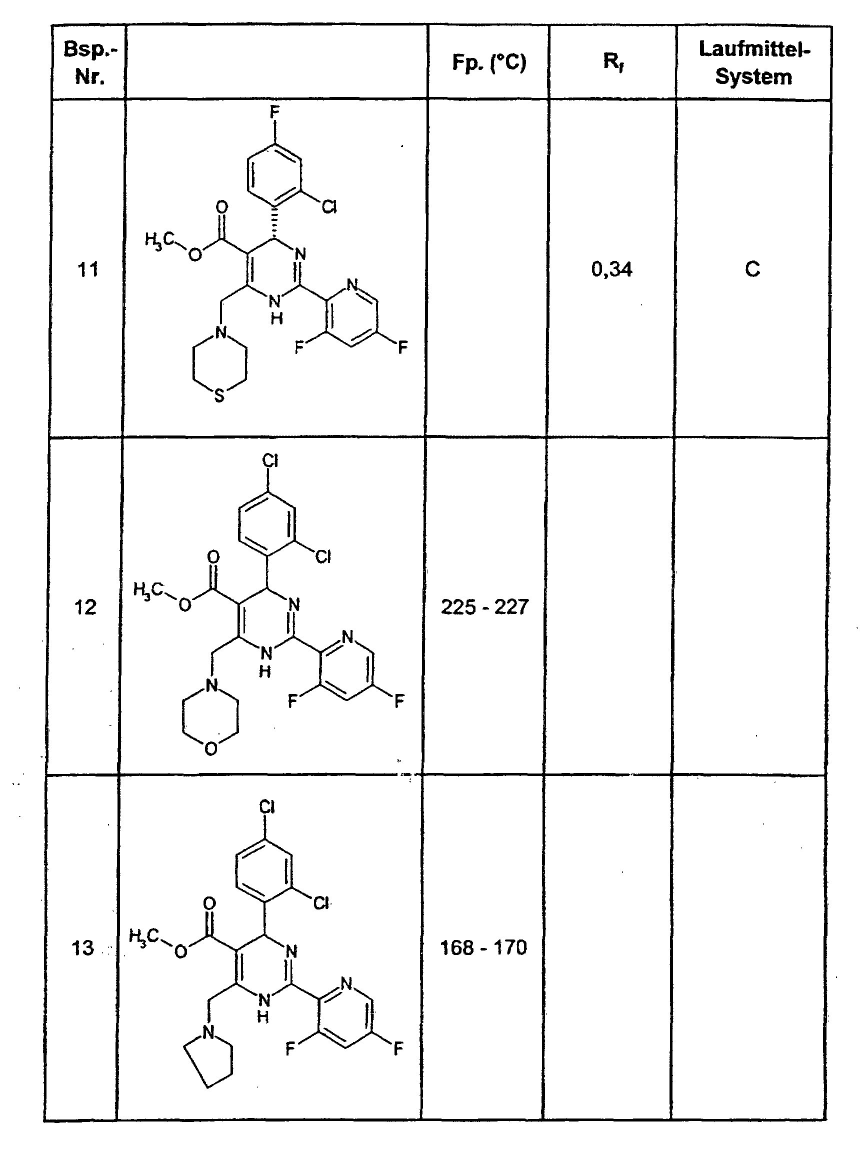

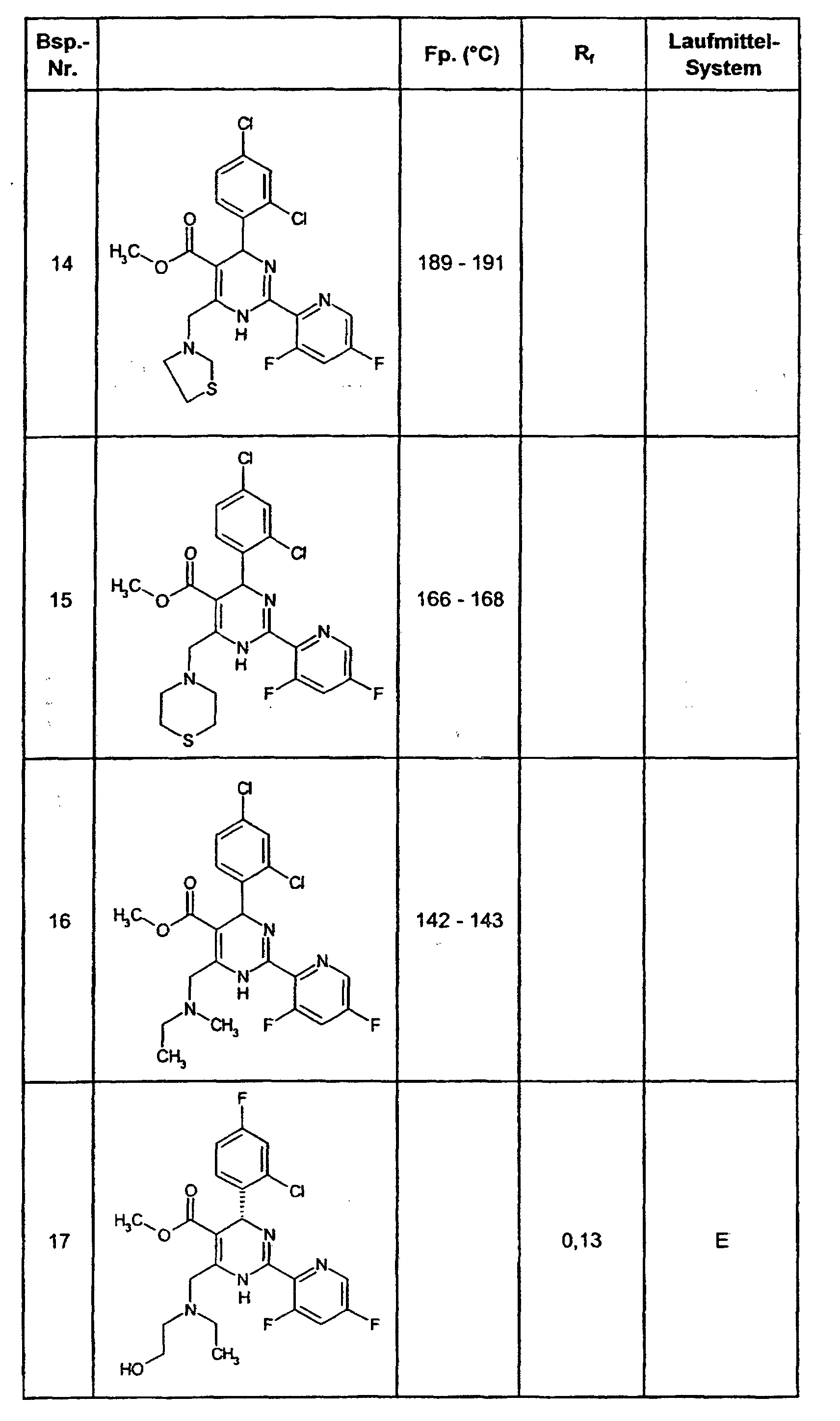

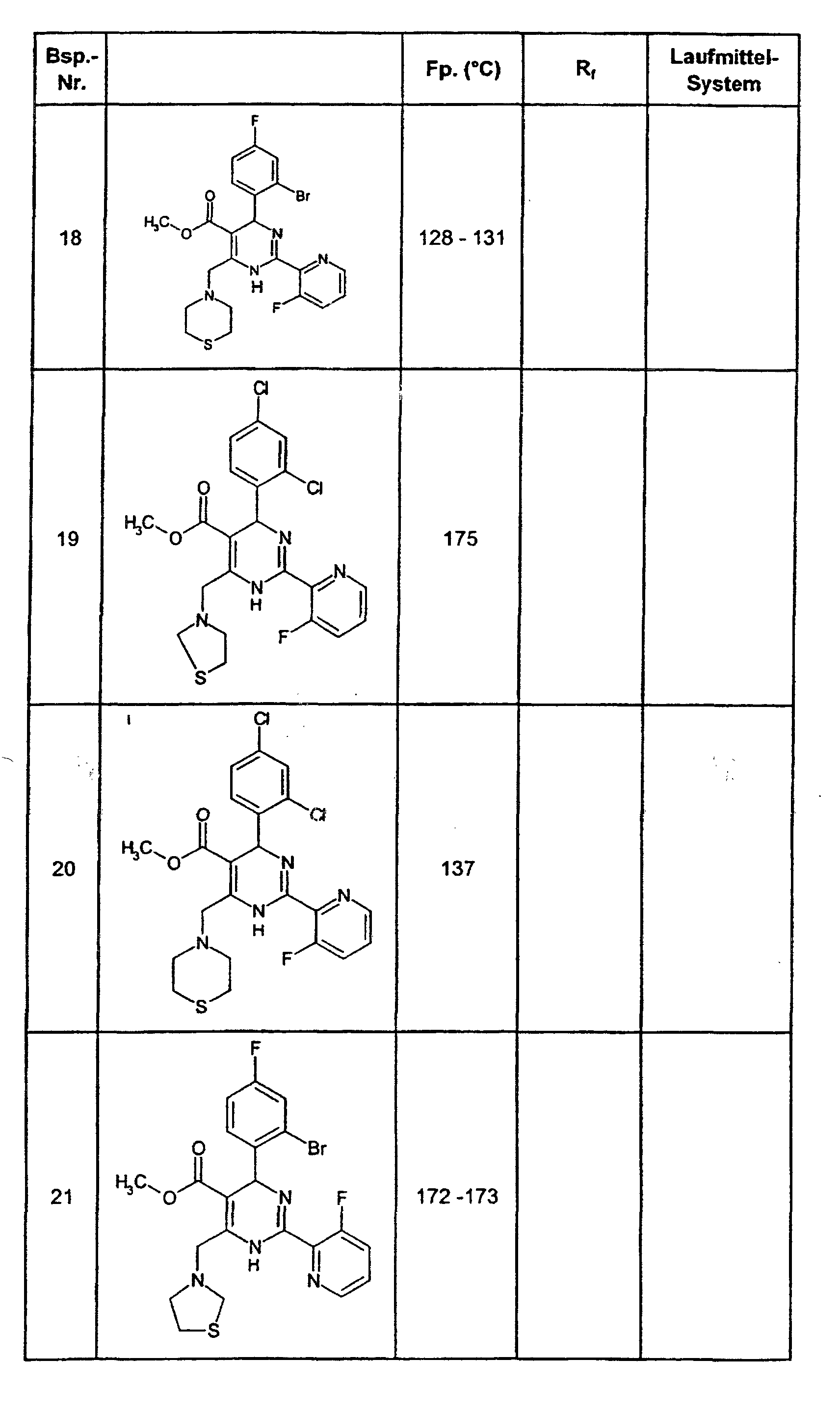

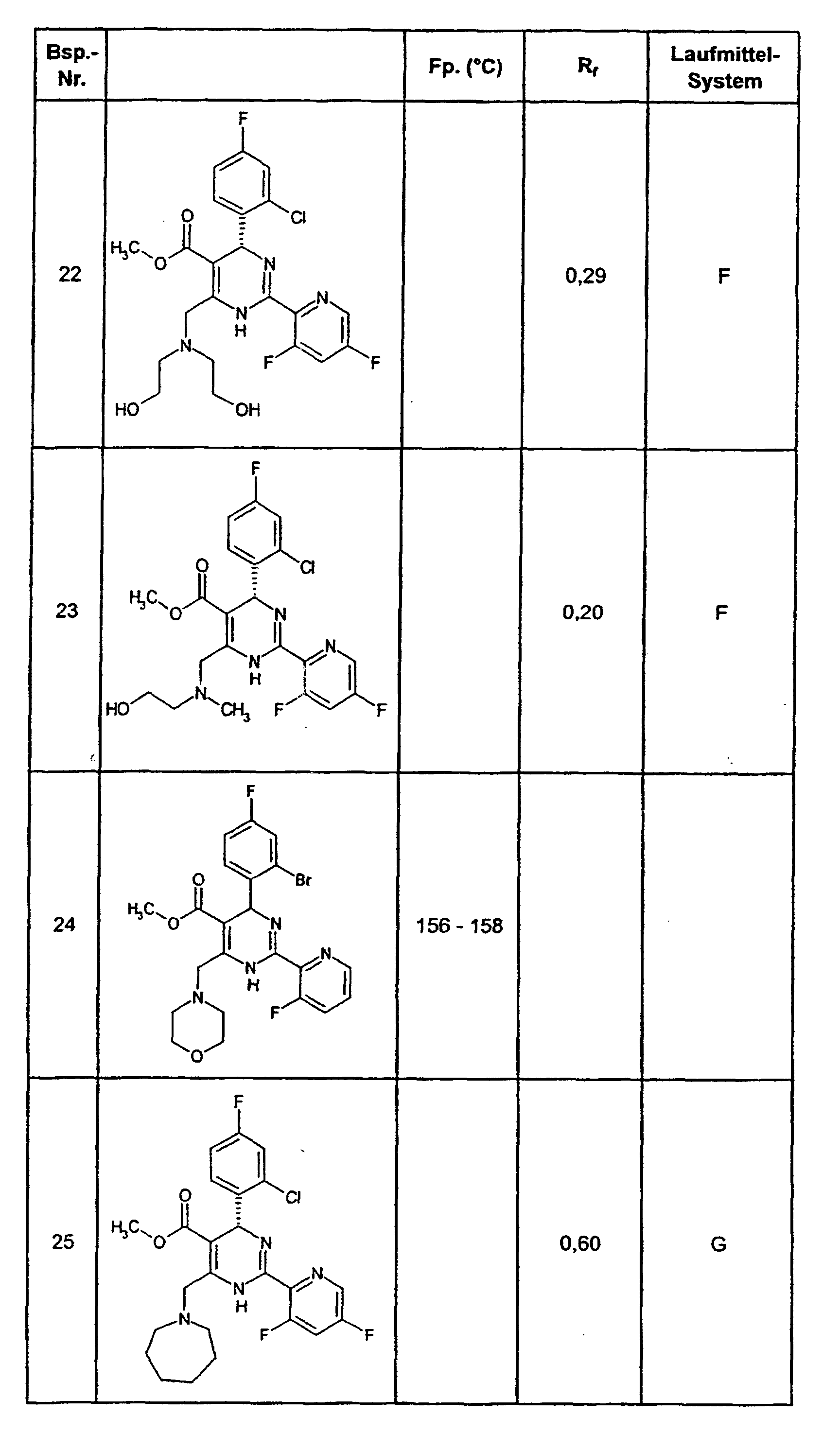

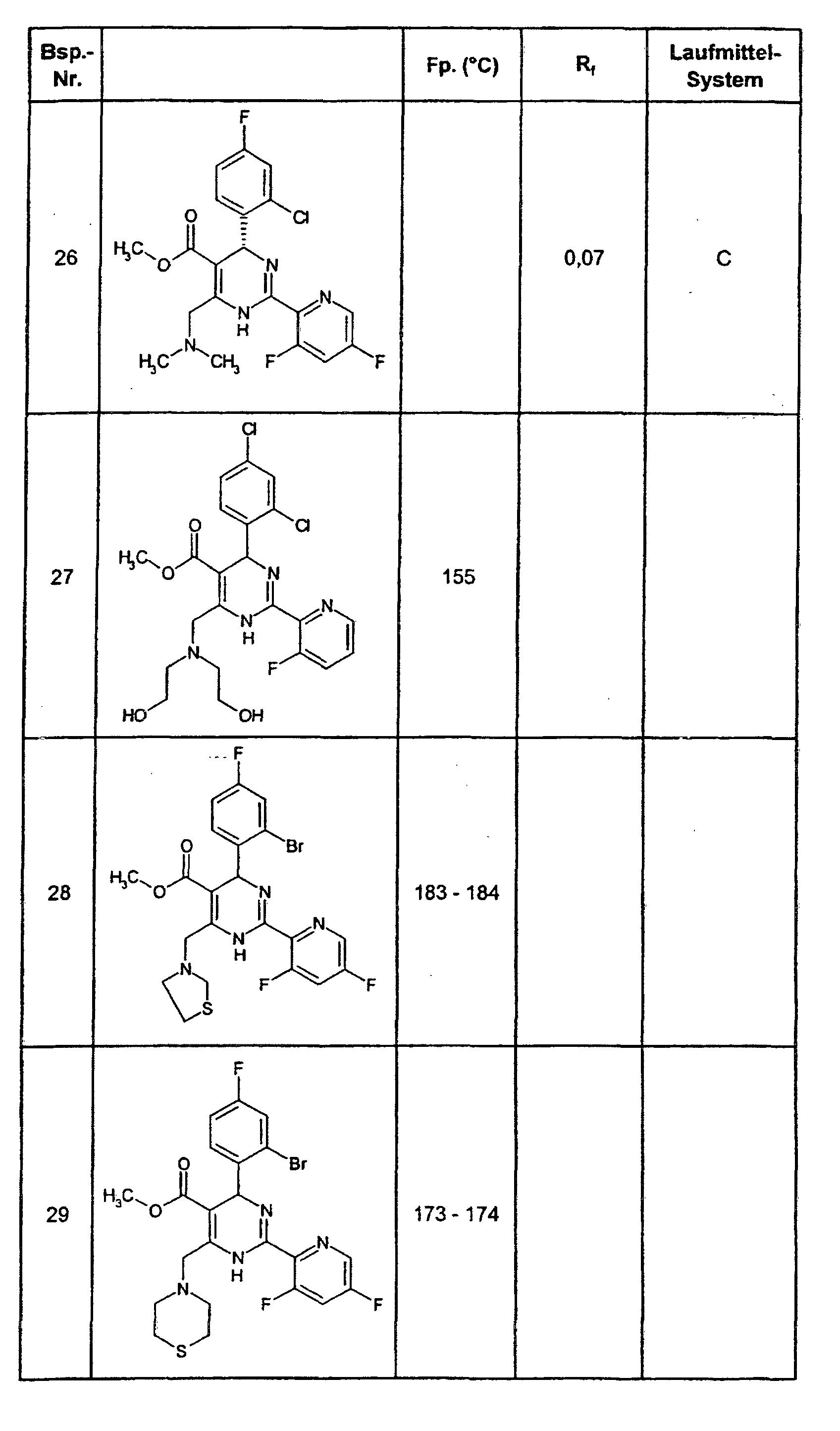

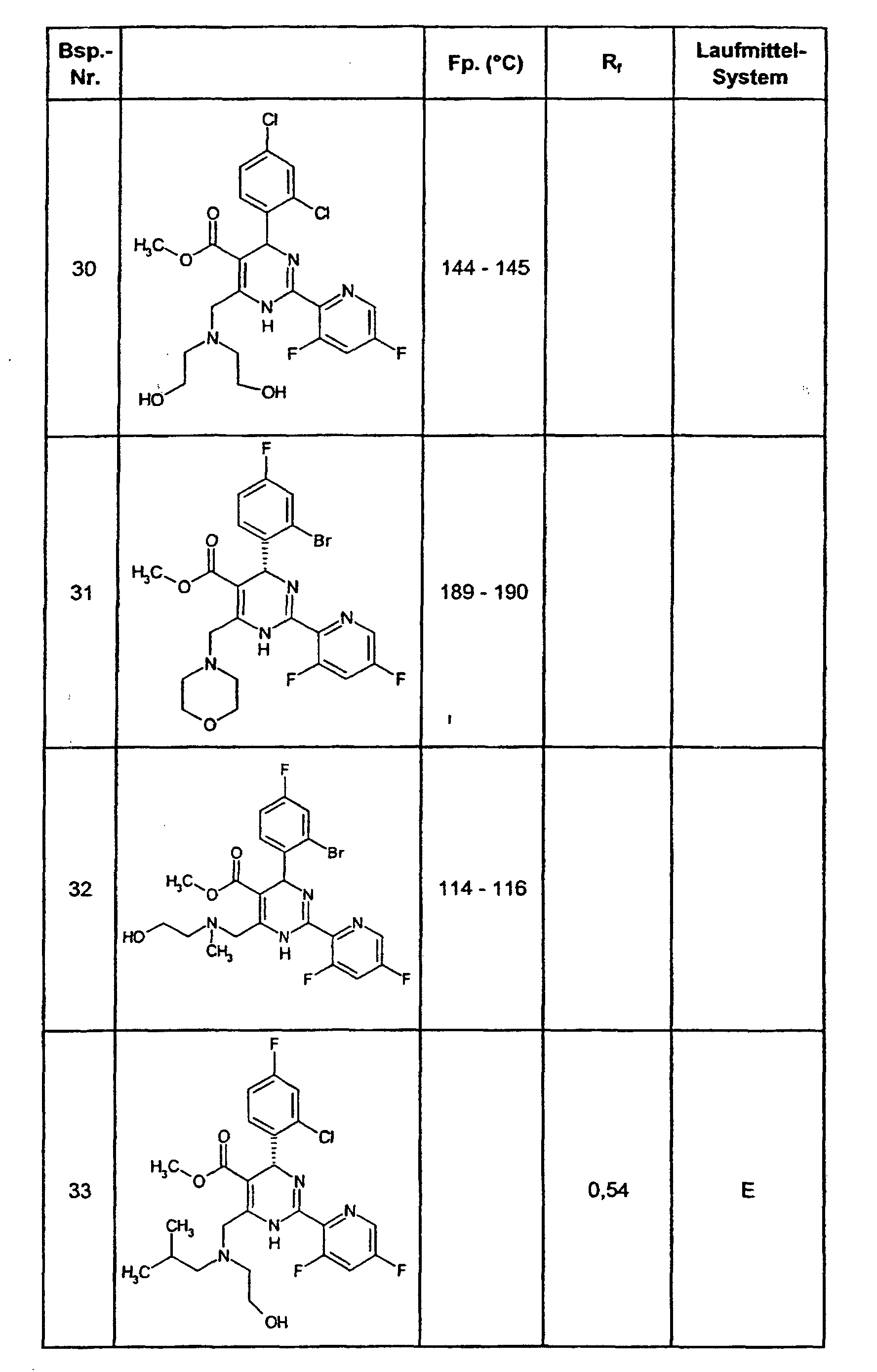

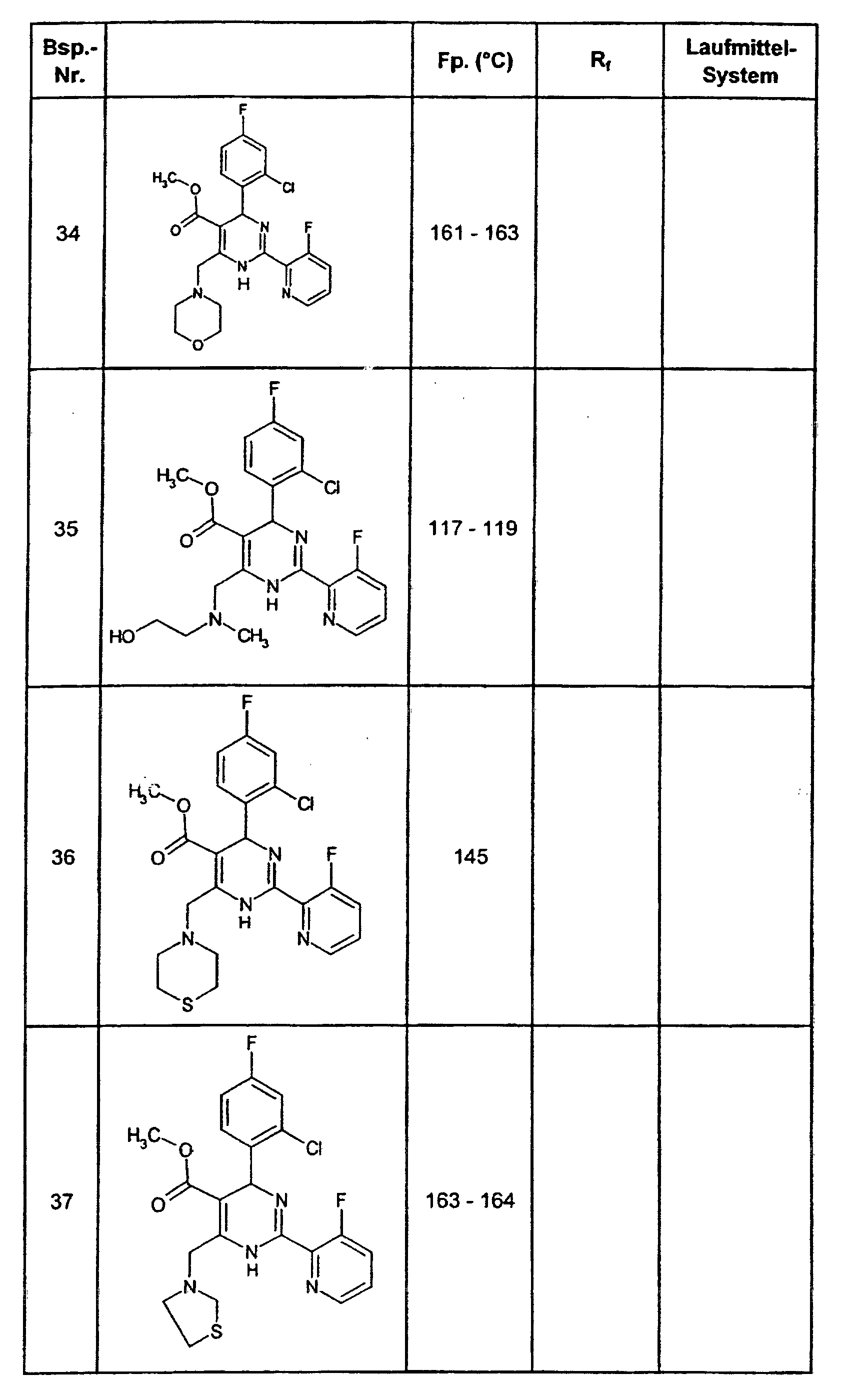

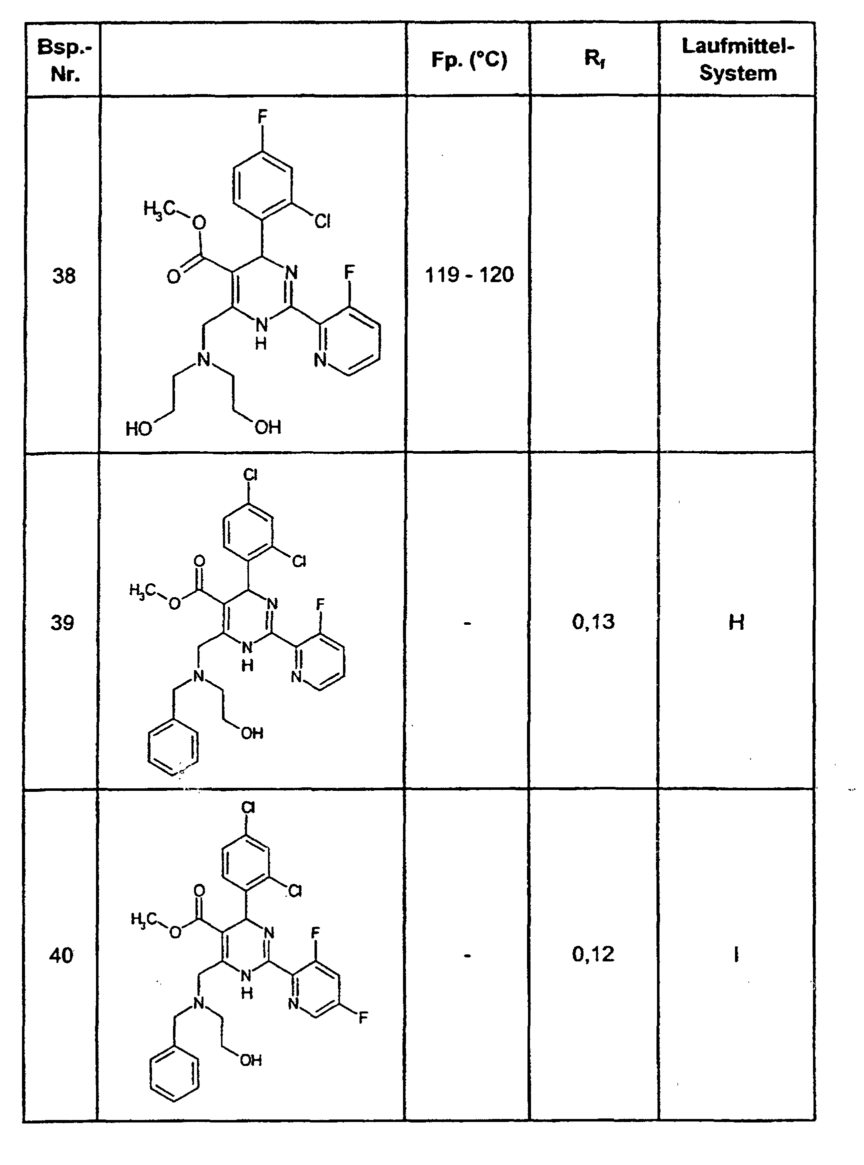

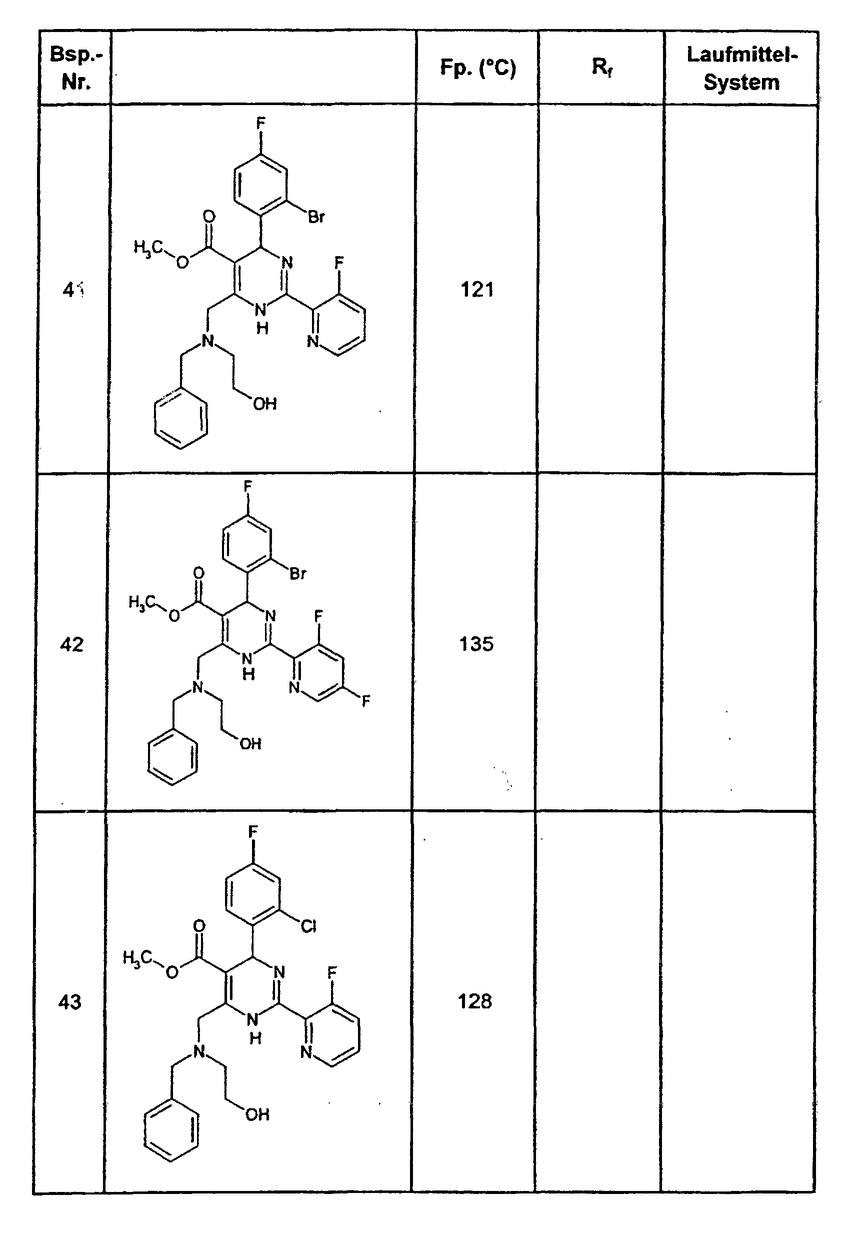

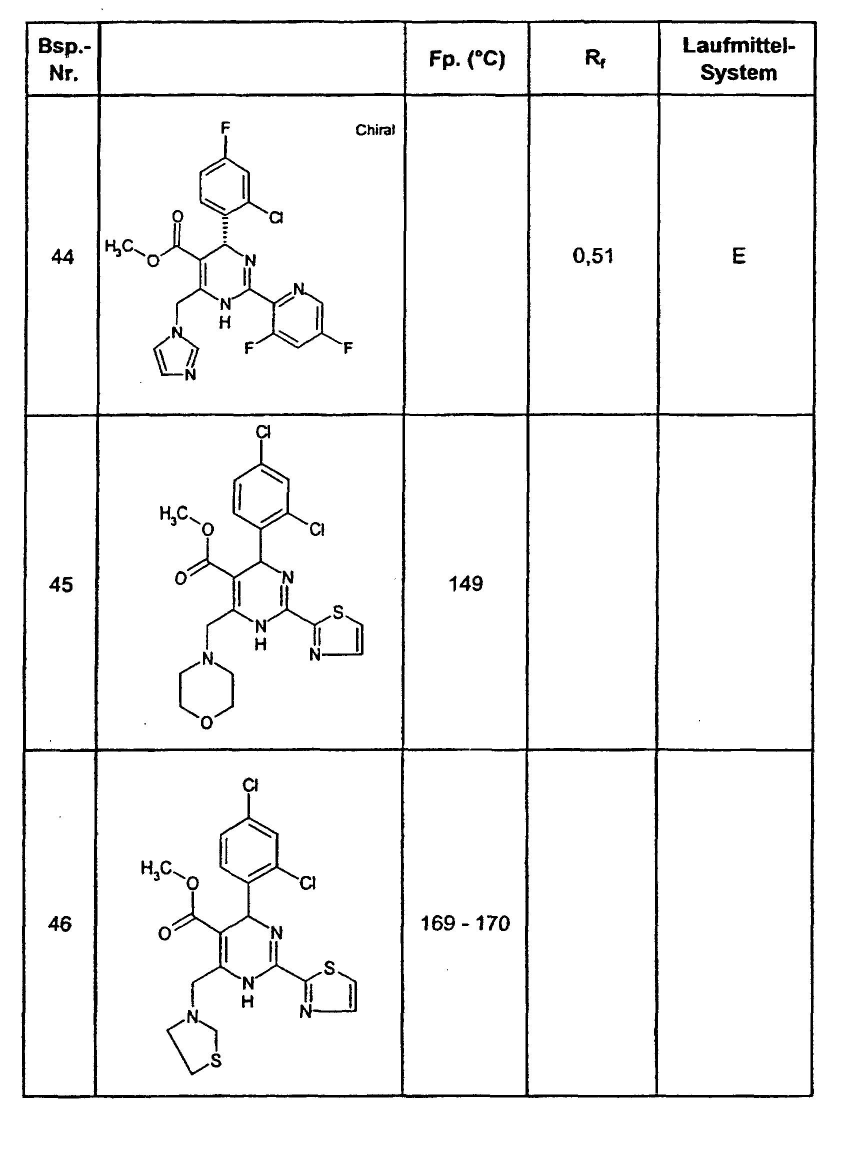

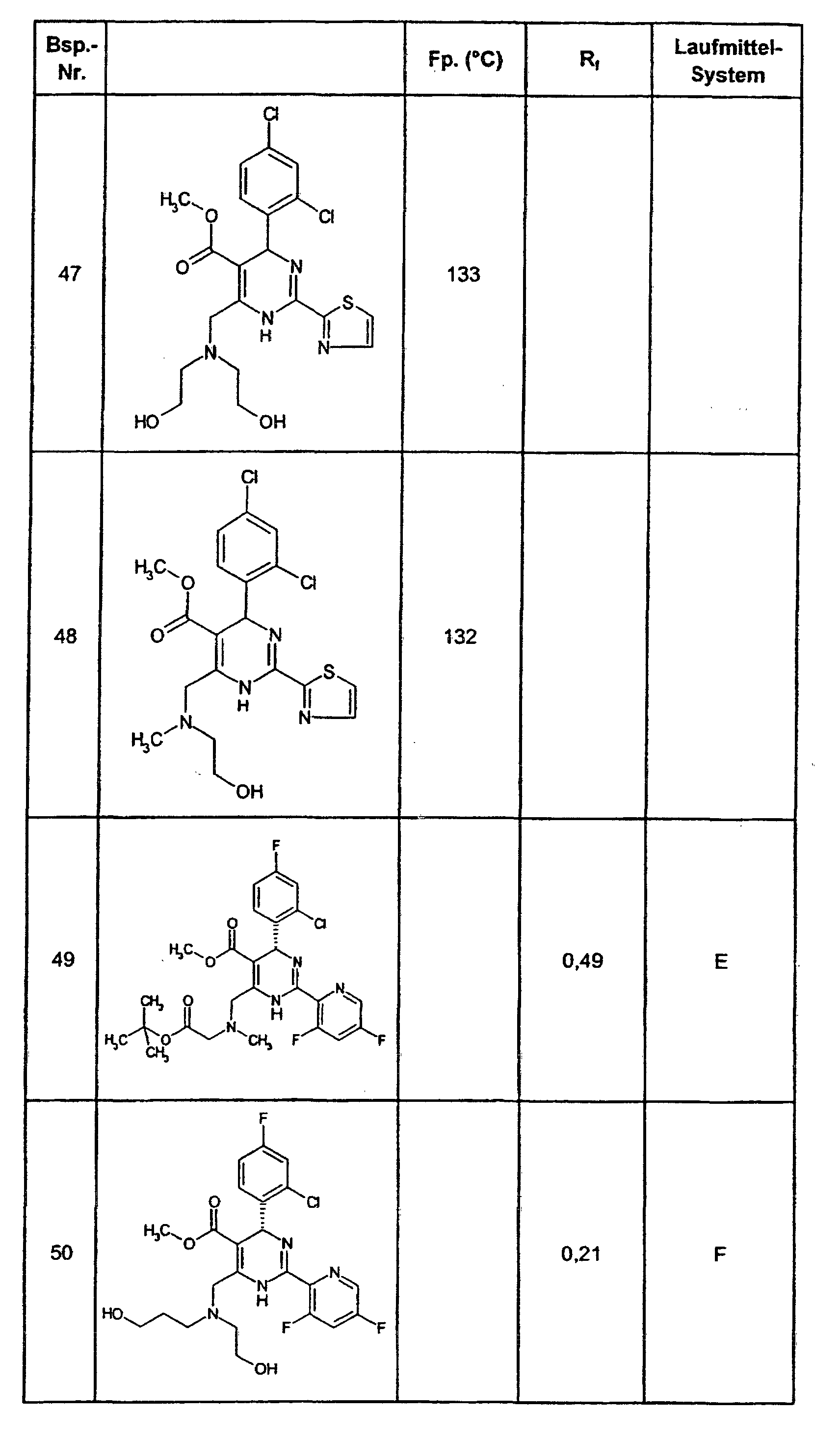

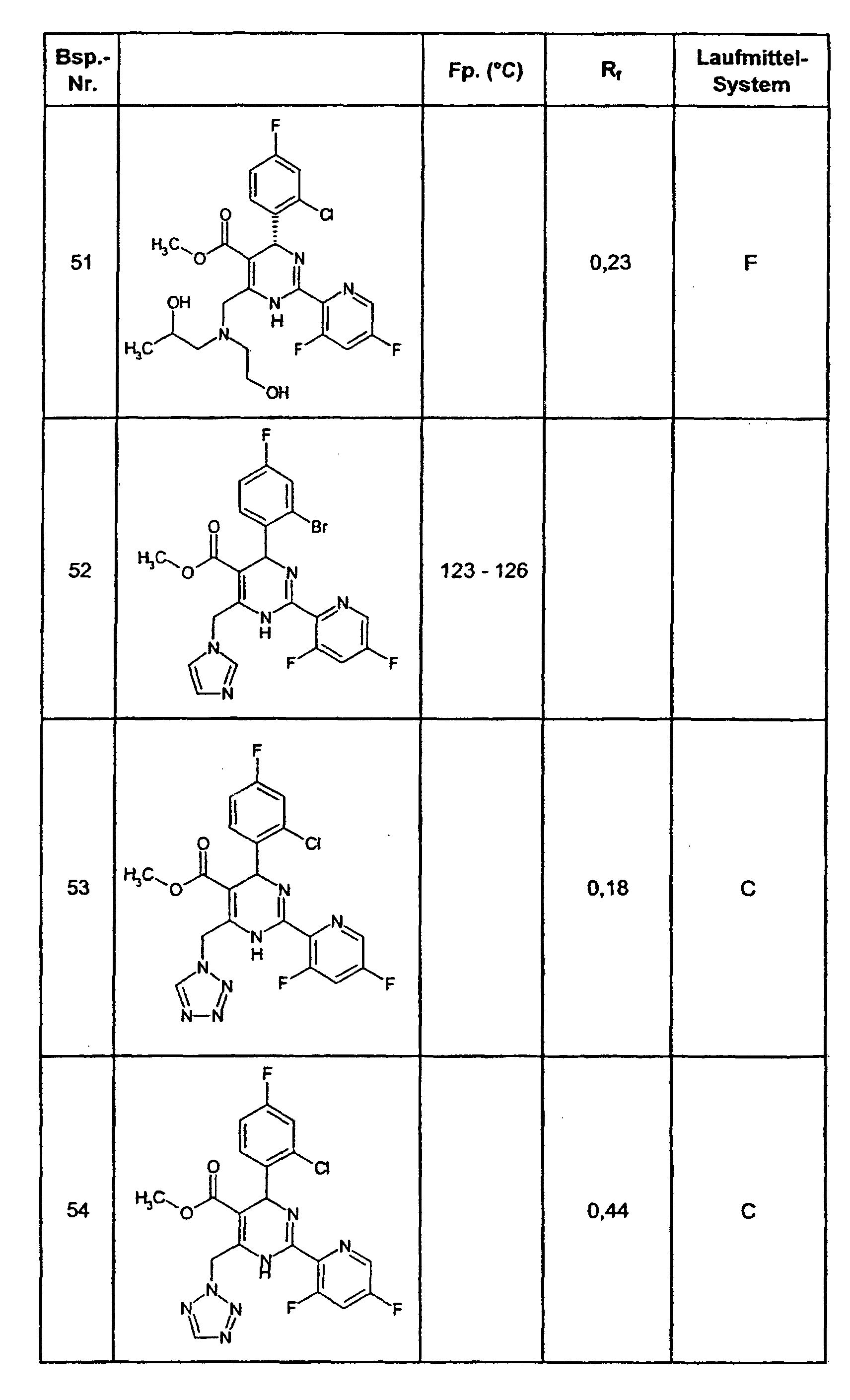

| **Laufmittel** | **Kürzel** |
|---|---|
| Cyclohexan/Ethylacetat = 7 : 3 | A |
| Cyclohexan/Ethylacetat = 8 : 2 | B |
| Cyclohexan/Ethylacetat = 1 : 1 | C |
| Cyclohexan/Ethylacetat = 7 : 3 + Tropfen NH₃ | D |
| Methylenchlorid/Methanol = 95 : 5 | E |
| Methylenchlorid/Methanol =9:1 | F |
| Toluol/Aceton = 1 : 1 | G |
| Toluol/Methanol =10 : 1 | H |
| Toluol/Ethylacetat = 4 : 1 | I |
| Methylenchlorid/Methanol =10:1 | J |
| Methylenchlorid/Methanol = 95 : 5 + Tropfen NH₃ | K |

Die Wirkdaten einiger erfindungsgemäßer Verbindungen werden nachfolgend aufgelistet:

| **Beispiel Nr.** | **IC**_{**50**} **(µM)** | **Tox**_{**50**} **(µM)** |
|---|---|---|
| 2 | 0,4 | 38 |
| 5 | 0,002 | 40 |
| 7 | 0,025 | 25 |
| 9 | 0,007 | 17 |
| 11 | 0,04 | >8 |
| 15 | 0,05 | 3 |
| 24 | 0,02 | 80 |
| 31 | 0,002 | 63 |
| 34 | 0,009 | 60 |
| 45 | 0,002 | 34 |

Die Behandlung der Hepatitis-B-Virus produzierenden HepG2.2.15-Zellen mit den erfindungsgemäßen Verbindungen führte überraschenderweise zu einer Reduktion intra- und/oder extrazellulärer viraler DNA.

## Patentansprüche

1. Verbindungen der Formel 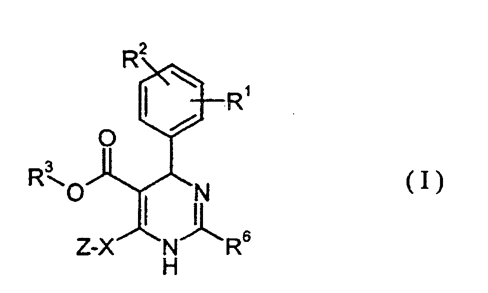 bzw. deren isomerer Form 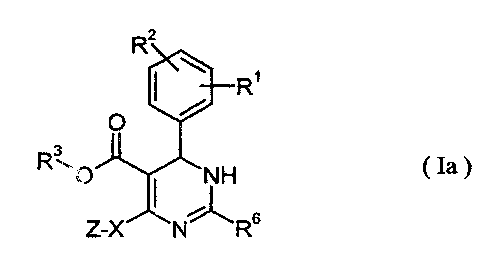 worin
R¹, R² unabhängig voneinander Wasserstoff, Fluor, Chlor oder Brom,
R³ C₁-C₄-Alkyl,
X eine Methylen- oder Ethylengruppe,
Z NR⁴R⁵ oder Pyridyl,
R⁴ C₁-C₄-Alkyl, das durch Hydroxy oder C₁-C₄-Alkoxycarbonyl substituiert sein kann, oder Benzyl,
R⁵ C₁-C₄-Alkyl, das durch Hydroxy substituiert sein kann,
oder
R⁴ und R⁵ gemeinsam mit dem Stickstoffatom, an das sie gebunden sind, einen Imidazolyl-, Triazolyl- oder Tetrazolyl-Ring oder einen Rest der Formel 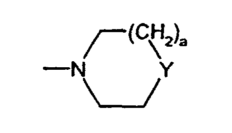
worin
a für Null oder 1 und
Y für CH₂, CH₂CH₂, -O- oder -S- stehen und
R⁶ Pyridyl, das ein- bis zweifach durch Fluor substituiert ist, oder Thiazolyl
bedeuten, und deren Salze.

2. Verbindungen nach Anspruch 1, worin
R⁴ und R⁵ gemeinsam mit dem Stickstoffatom, an das sie gebunden sind, einen Morpholinyl- oder Thiomorpholinyl-Ring bilden,
und deren Salze.

3. Verbindungen nach Anspruch 1, worin
R¹, R² unabhängig voneinander Fluor, Chlor oder Brom
bedeuten, und deren Salze.

4. Verbindungen nach Anspruch 1, worin
R¹, R² unabhängig voneinander Fluor, Chlor oder Brom bedeuten und
R⁴ und R⁵ gemeinsam mit dem Stickstoffatom, an das sie gebunden sind, einen Morpholinyl- oder Thiomorpholinyl-Ring bilden,
und deren Salze.

5. Verbindungen der Beispiele 5, 7, 9, 11, 12, 15, 24, 31, 34, 45.

6. Verfahren zur Herstellung der Verbindungen nach Ansprüchen 1 bis 5, wonach man
[A] Verbindungen der Formel 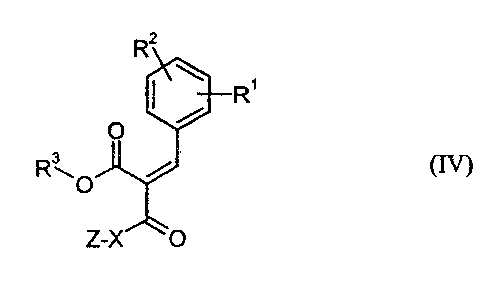 worin
R¹ bis R³, X und Z die in Ansprüchen 1 bis 5 angegebenen Bedeutungen besitzen,
mit Amidinen der Formel 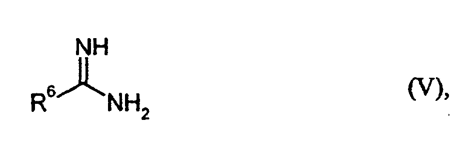 worin
R⁶ die in Ansprüchen 1 bis 5 angegebene Bedeutung hat,
oder deren Salzen umsetzt oder
[B] Verbindungen der Formel 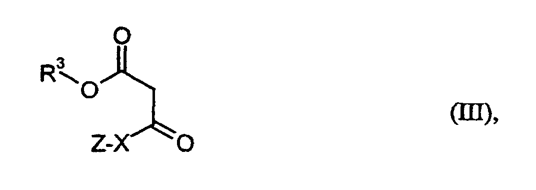 worin
R³, X und Z die in Ansprüchen 1 bis 5 angegebenen Bedeutungen besitzen,
in einem einstufigen Verfahren mit Aldehyden der Formel 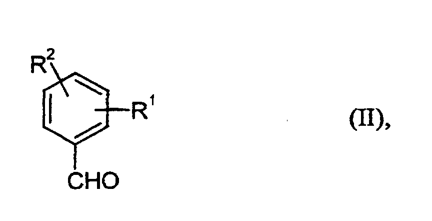 worin
R¹ und R² die in Ansprüchen 1 bis 5 angegebenen Bedeutungen besitzen,
und Amidinen der Formel 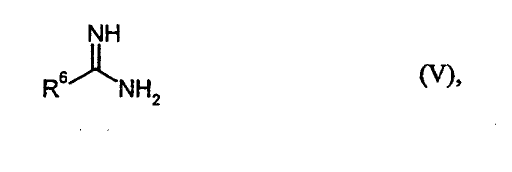 worin
R⁶ die in Ansprüchen 1 bis 5 angegebene Bedeutung hat,
oder deren Salzen umsetzt oder auch
[C] sofern X in Formel (I) für eine Methylengruppe steht, Verbindungen der Formel 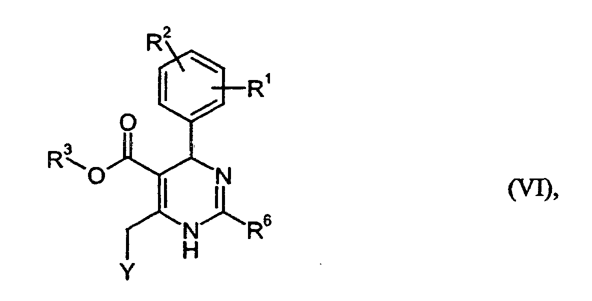 worin
R¹ bis R³ und R⁶ die in Ansprüchen 1 bis 5 angegebenen Bedeutungen besitzen und
Y für eine nukleophil austauschbare Gruppe steht,
mit Verbindungen der Formel 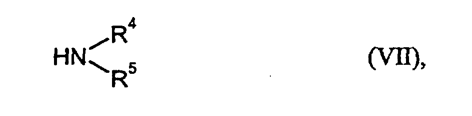 worin
R⁴ und R⁵ die in Ansprüchen 1 bis 5 angegebenen Bedeutungen besitzen,
umsetzt oder auch
[D] sofern X in Formel (I) für eine Ethylengruppe steht, Verbindungen der Formel 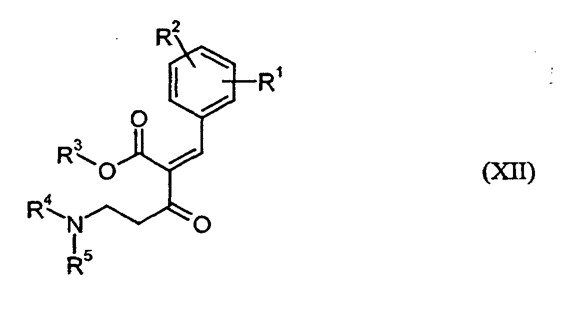
worin
R¹ bis R⁵ die in Ansprüchen 1 bis 5 angegebenen Bedeutungen besitzen, mit Amidinen der Formel 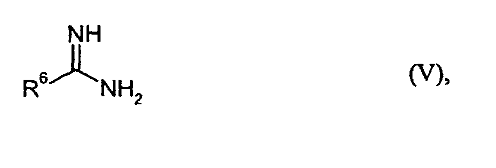
worin
R⁶ die in Ansprüchen 1 bis 5 angegebene Bedeutung hat,
oder deren Salzen umsetzt.

7. Verfahren zur Herstellung der Verbindungen nach Ansprüchen 1 bis 5, wonach man
[A] Aldehyde der Formel 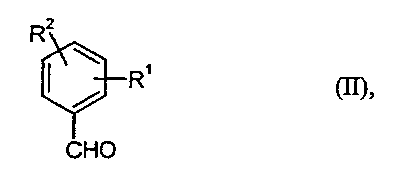 worin
R¹ und R² die in Ansprüchen 1 bis 5 angegebenen Bedeutungen besitzen,
zunächst mit β-Ketoestem der Formel 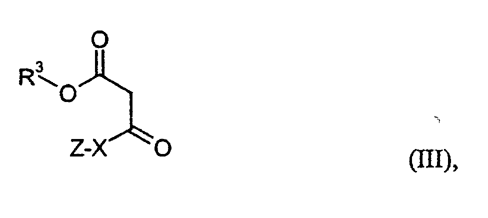 worin
R³, X und Z die in Ansprüchen 1 bis 5 angegebenen Bedeutungen besitzen,
in Benzyliden-Verbindungen der Formel 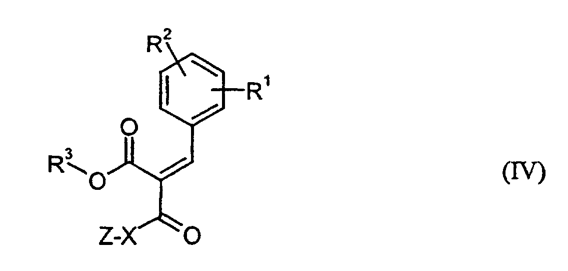 überführt und diese dann mit Amidinen der Formel 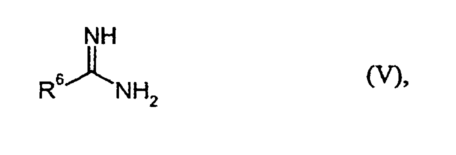 worin
R⁶ die in Ansprüchen 1 bis 5 angegebene Bedeutung hat,
oder deren Salzen umsetzt oder
[B] Verbindungen der Formel (III) in einem einstufigen Verfahren mit Aldehyden (II) und Amidinen (V) oder deren Salzen umsetzt oder auch
[C] sofern X in Formel (I) für eine Methylengruppe steht, Verbindungen der Formel 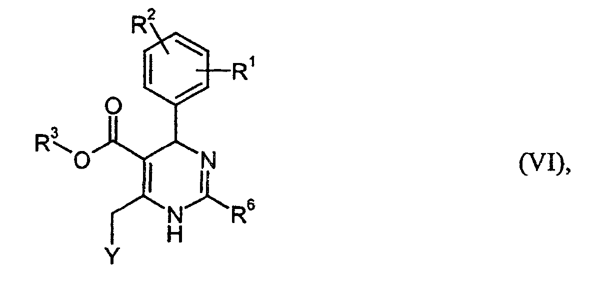 worin
R¹ bis R³ und R⁶ die in Ansprüchen 1 bis 5 angegebenen Bedeutungen besitzen und
Y für eine nukleophil austauschbare Gruppe steht,
mit Verbindungen der Formel 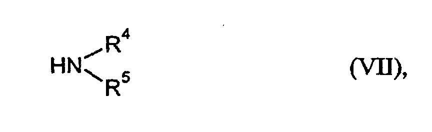 worin
R⁴ und R⁵ die in Ansprüchen 1 bis 5 angegebenen Bedeutungen besitzen, umsetzt oder auch
[D] sofern X in Formel (I) für eine Ethylengruppe steht, Verbindungen der Formel 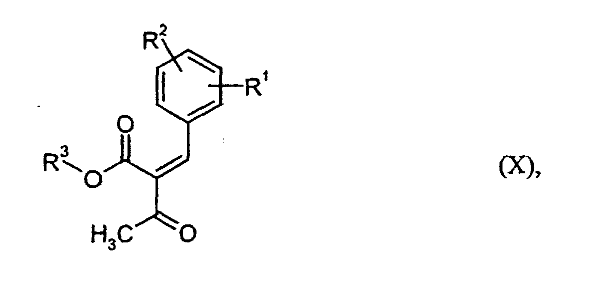
worin
R¹ bis R³ die in Ansprüchen 1 bis 5 angegebenen Bedeutungen besitzen,
mit Immoniumsalzen der Formel 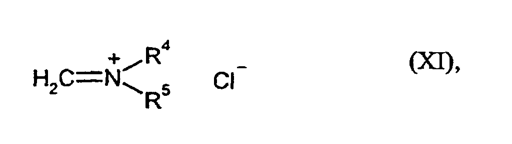 worin
R⁴ und R⁵ die in Ansprüchen 1 bis 5 angegebenen Bedeutungen besitzen,
in Verbindungen der Formel 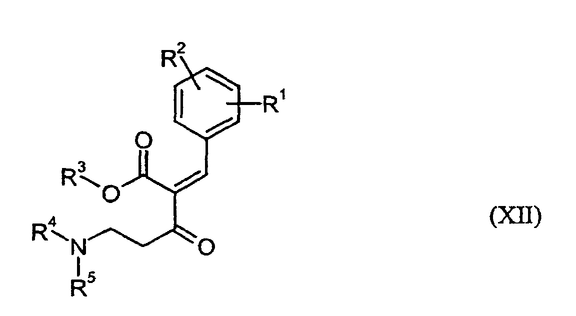 überführt und diese dann mit Amidinen der Formel (V) oder deren Salzen umsetzt.

8. Verbindungen der Formel 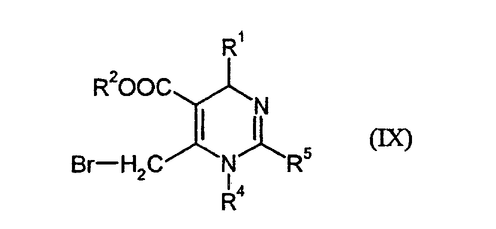 worin
R¹, R², R⁴ und R⁵ die in den Ansprüchen 1 bis 5 angegebenen Bedeutungen besitzen.

9. Verbindungen nach Ansprüchen 1 bis 5 zur Bekämpfung von Erkrankungen.

10. Arzneimittel, enthaltend mindestens eine Verbindung nach Ansprüchen 1 bis 5 und gegebenenfalls weitere pharmazeutische Wirkstoffe.

11. Verwendung von Verbindungen der Ansprüche 1 bis 5 zur Herstellung eines Arzneimittels zur Behandlung und Prophylaxe von Viruserkrankungen.

12. Verwendung von Verbindungen der Ansprüche 1 bis 5 zur Herstellung eines Arzneimittels zur Behandlung und Prophylaxe von Hepatitis-B-Infektionen.

13. Kombinationen
A) mindestens eines Dihydropyrimidins nach Ansprüchen 1 bis 5,
B) mindestens eines von A verschiedenen HBV-antiviralen Mittels und gegebenenfalls
C) mindestens eines Immunmodulators.

14. Kombinationen nach Anspruch 13, worin die Komponente B ein HBV-Polymerase-Inhibitor ist.

15. Kombinationen nach Anspruch 13, worin die Komponente B Lamivudin ist.

16. Kombinationen nach Anspruch 13, worin die Komponente B aus den Verbindungen der Formel 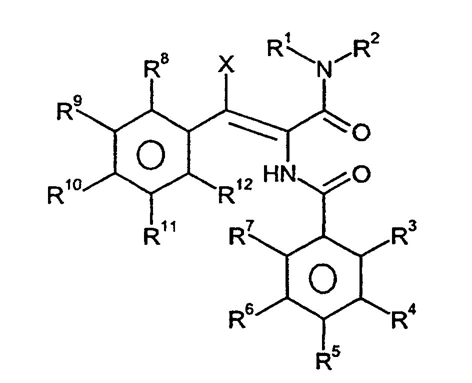 worin
R¹ und R² unabhängig voneinander C₁-C₄-Alkyl bedeuten oder zusammen mit dem Stickstoffatom, an dem sie stehen, einen Ring mit 5 bis 6 Ringatomen, die Kohlenstoff und/oder Sauerstoff umfassen, bilden,
R³-R¹² unabhängig voneinander Wasserstoff, Halogen, C₁-C₄-Alkyl, gegebenenfalls substituiertes C₁-C₄-Alkoxy, Nitro, Cyano oder Trifluormethyl,
R¹³ Wasserstoff, C₁-C₄-Alkyl, C₁-C₇-Acyl oder Aralkyl und
X Halogen oder gegebenenfalls substituiertes C₁-C₄-Alkyl bedeuten,
und deren Salzen ausgewählt ist.

17. Kombinationen nach Anspruch 16, worin
X Chlor, A 1-Piperidinyl und Y und Z jeweils Phenyl bedeuten.

18. Kombinationen nach Ansprüchen 13 bis 17, worin der Immunmodulator C Interferone enthält.

19. Kombinationen von A) Dihydropyrimidin nach Ansprüchen 1 bis 5, B) Lamivudin und gegebenenfalls C) Interferon.

20. Verfahren zur Herstellung der Kombinationen nach Ansprüchen 13 bis 19, **dadurch gekennzeichnet, dass** man die Komponenten A, B und gegebenenfalls C in geeigneter Weise kombiniert oder herrichtet.

21. Kombinationen nach Ansprüchen 13 bis 19 zur Bekämpfung von Erkrankungen.

22. Arzneimittel, enthaltend mindestens eine Kombination gemäß Ansprüchen 13 bis 19 und gegebenenfalls weitere pharmazeutische Wirkstoffe.

23. Verwendung von Kombinationen der Ansprüche 13 bis 19 zur Herstellung eines Arzneimittels zur Behandlung und Prophylaxe von Viruserkrankungen.

24. Verwendung von Kombinationen der Ansprüche 13 bis 19 zur Herstellung eines Arzneimittels zur Behandlung und Prophylaxe von Hepatitis-B-Infektionen.

## Claims

1. Compounds of the formula 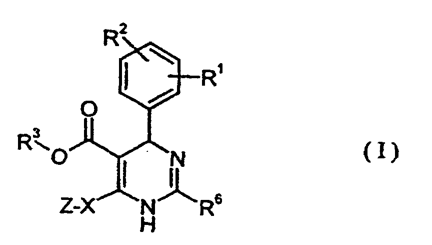 and the isomeric form thereof 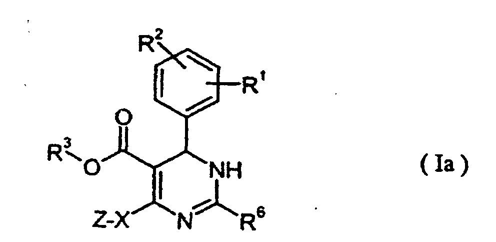 in which
R¹, R² are, independently of one another, hydrogen, fluorine, chlorine or bromine,
R³ is C₁-C₄-alkyl,
X is a methylene or ethylene group,
Z is NR⁴R⁵ or pyridyl,
R⁴ is C₁-C₄-alkyl which may be substituted by hydroxyl or C₁-C₄-alkoxycarbonyl, or is benzyl,
R⁵ is C₁-C₄-alkyl which may be substituted by hydroxyl,
or
R⁴ and R⁵ together with the nitrogen atom to which they are bonded are an imidazolyl, triazolyl or tetrazolyl ring or a radical of the formula 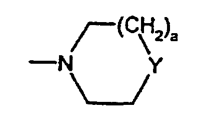
in which
a is zero or 1 and
Y is CH₂, CH₂CH₂, -O- or -S-, and
R⁶ is pyridyl which is substituted once to twice by fluorine, or is thiazolyl,
and the salts thereof.

2. Compounds according to Claim 1, in which
R⁴ and R⁵ together with the nitrogen atom to which they are bonded form a morpholinyl or thiomorpholinyl ring,
and the salts thereof.

3. Compounds according to Claim 1, in which
R¹, R² are, independently of one another, fluorine, chlorine or bromine,
and the salts thereof.

4. Compounds according to Claim 1, in which
R¹, R² are, independently of one another, fluorine, chlorine or bromine, and
R⁴ and R⁵ together with the nitrogen atom to which they are bonded form a morpholinyl or thiomorpholinyl ring,
and the salts thereof.

5. Compounds of Example 5, 7, 9, 11, 12, 15, 24, 31, 34, 45.

6. Process for preparing the compounds according to Claims 1 to 5, by
[A] reacting compounds of the formula 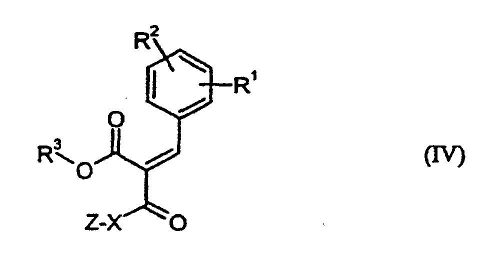 in which
R¹ to R³, X and Z have the meanings indicated in Claims 1 to 5,
with amidines of the formula 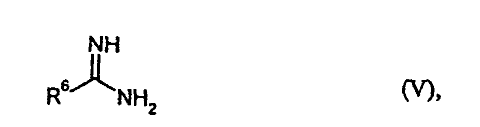 in which
R⁶ has the meaning indicated in Claims 1 to 5,
or the salts thereof, or
[B] reacting compounds of the formula 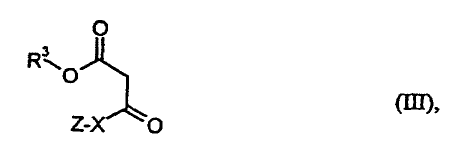 in which
R³, X and Z have the meanings indicated in Claims 1 to 5,
in a one-stage process with aldehydes of the formula 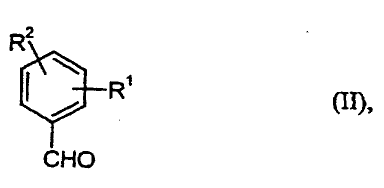 in which
R¹ and R² have the meanings indicated in Claims 1 to 5,
and amidines of the formula 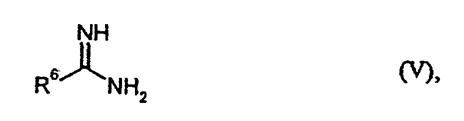 in which
R⁶ has the meanings indicated in Claims 1 to 5,
or the salts thereof, or else
[C] where X in formula (I) is a methylene group, reacting compounds of the formula 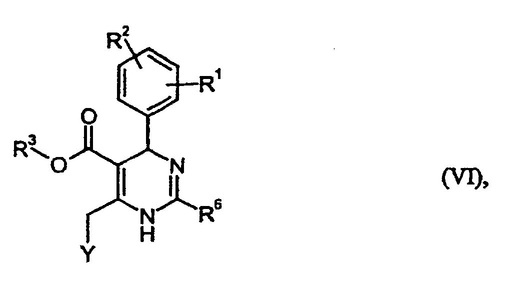 in which
R¹ to R³ and R⁶ have the meanings indicated in Claims 1 to 5, and
Y . is a nucleophilically replaceable group,
with compounds of the formula 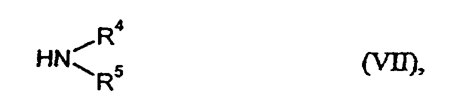 in which
R⁴ and R⁵ have the meanings indicated Claims 1 to 5, or else
[D] where X in formula (I) is an ethylene group, reacting compounds of the formula 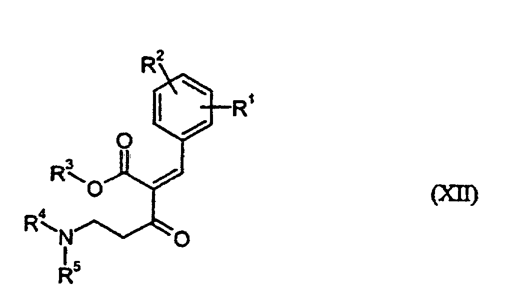
in which
R¹ to R⁵ have the meanings indicated in Claims 1 to 5, with amidines of the formula 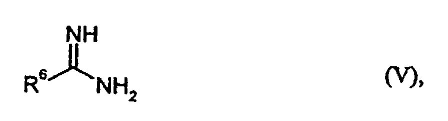
in which
R⁶ has the the meanings indicated in Claims 1 to 5,
or the salts thereof.

7. Process for preparing the compounds according to Claims 1 to 5, by
[A] firstly converting aldehydes of the formula 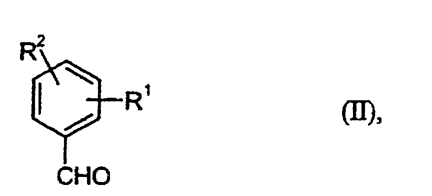 in which
R¹ and R² have the meanings indicated in Claims 1 to 5,
with β-keto esters of the formula 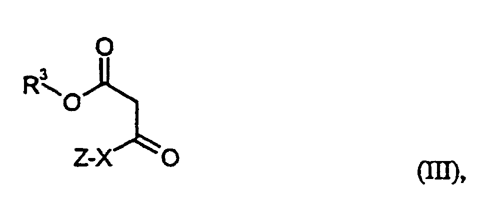 in which
R³, X and Z have the meanings indicated in Claims 1 to 5,
into benzylidene compounds of the formula 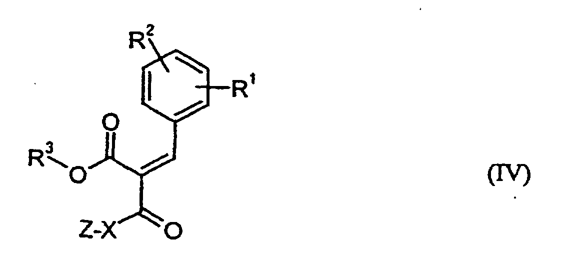 and then reacting the latter with amidines of the formula 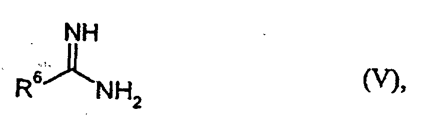 in which
R⁶ has the meaning indicated in Claims 1 to 5,
or the salts thereof, or
[B] reacting compounds of the formula (III) in a one-stage process with aldehydes (II) and amidines (V) or the salts thereof, or else
[C] where X in formula (I) is a methylene group, reacting compounds of the formula 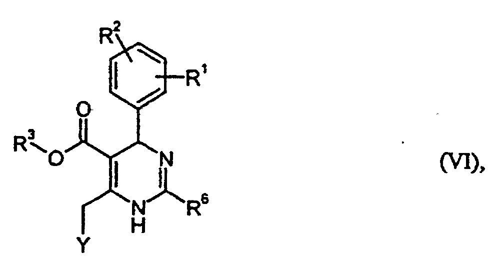 in which
R¹ to R³ and R⁶ have the meanings indicated in Claims 1 to 5, and
Y is a nucleophilically replaceable group,
with compounds of the formula 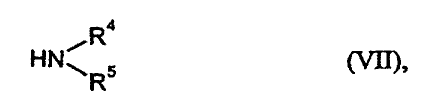 in which
R⁴ and R⁴ have the meanings indicated in Claims 1 to 5, or else
[D] where X in formula (1) is an ethylene group, converting compounds of the formula 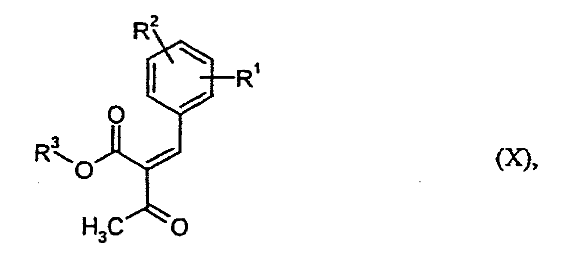
in which
R¹ to R³ have the meanings indicated in Claims 1 to 5,
with immonium salts of the formula 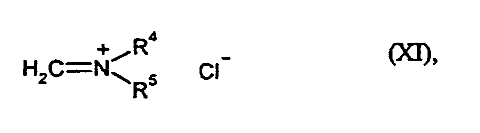 in which
R⁴ and R⁵ have the meanings indicated in Claims 1 to 5,
into compounds of the formula 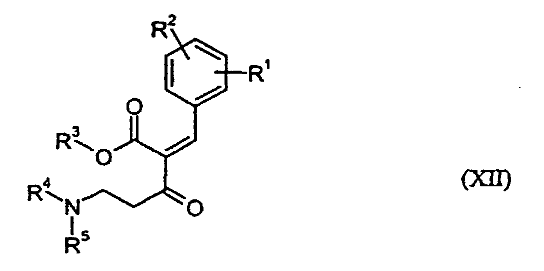 and then reacting the latter with amidines of the formula (V) or the salts thereof

8. Compounds of the formula 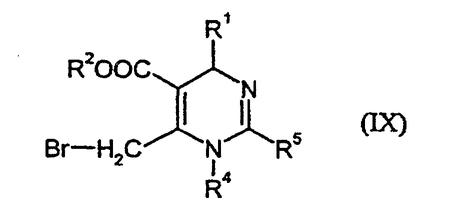 in which
R¹, R², R⁴ and R⁵ have the meanings indicated in Claims 1 to 5.

9. Compounds according to any of Claims 1 to 5 for controlling diseases.

10. Medicament comprising at least one compound according to Claims 1 to 5 and, where appropriate, other active pharmaceutical ingredients.

11. Use of compounds of Claims 1 to 5 for producing a medicament for the treatment and prophylaxis of viral diseases.

12. Use of compounds of Claims 1 to 5 for producing a medicament for the treatment and prophylaxis of heptatitis B infections.

13. Combinations of
A) at least one dihydropyrimidine according to Claims 1 to 5,
B) at least one HBV antiviral agent different from A and, where appropriate,
C) at least one immunomodulator.

14. Combinations according to Claim 13, in which component B is an HBV polymerase inhibitor.

15. Combinations according to Claim 13, in which component B is lamivudine.

16. Combinations according to Claim 13, in which component B is selected from compounds of the formula 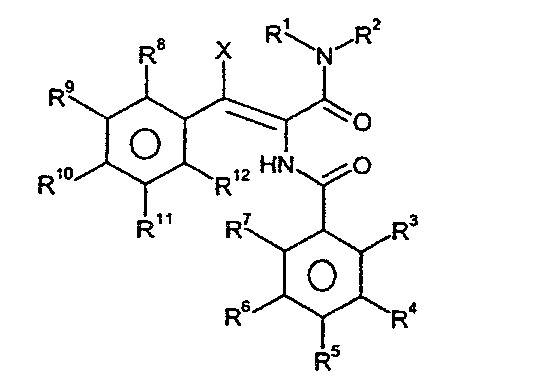 in which
R¹ and R² are, independently of one another, C₁-C₄-alkyl or, together with the nitrogen atom on which they are located, form a ring having 5 to 6 ring atoms which comprise carbon and/or oxygen,
R³-R¹² are, independently of one another, hydrogen, halogen, C₁-C₄-alkyl, optionally substituted C₁-C₄-alkoxy, nitro, cyano or trifluoromethyl,
R¹³ is hydrogen, C₁-C₄-alkyl, C₁-C₇-acyl or aralkyl and
X is halogen or optionally substituted C₁-C₄-alkyl,
and the salts thereof.

17. Combinations according to Claim 16, in which
X is chlorine, A is 1-piperidinyl and Y and Z are each phenyl.

18. Combinations according to Claims 13 to 17, in which the immunomodulator . C comprises interferons.

19. Combinations of A) dihydropyrimidine according to Claims 1 to 5, B) lamivudine and, where appropriate, C) interferon.

20. Process for producing the combinations according to Claims 13 to 19, **characterized in that** components A, B and, where appropriate, C are combined or prepared in a suitable way.

21. Combinations according to Claims 13 to 19 for controlling diseases.

22. Medicament comprising at least one combination according to Claims 13 to 19 and, where appropriate, other active pharmaceutical ingredients.

23. Use of combinations of Claims 13 to 19 for producing a medicament for the treatment and prophylaxis of viral diseases.

24. Use of combinations of Claims 13 to 19 for producing a medicament for the treatment and prophylaxis of hepatitis B infections.

## Revendications

1. Composés de formule 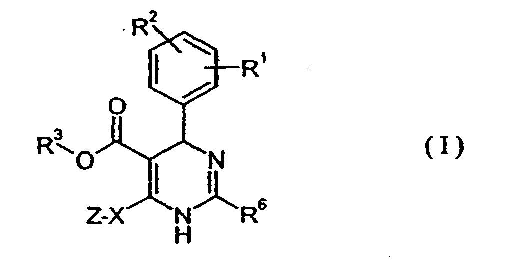 et leurs formes isomères 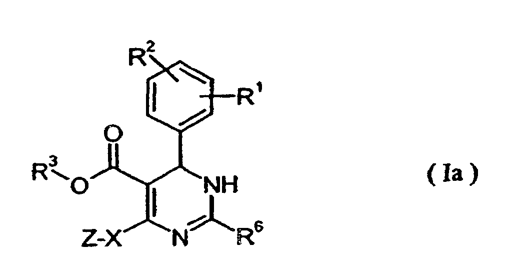 formules dans lesquelles
R¹, R² représentent indépendamment l'un de l'autre l'hydrogène, le fluor, le chlore ou le brome,
R³ est un reste alkyle en C₁ à C₄,
X est un groupe méthylène ou éthylène,
Z est un groupe NR⁴R⁵ ou un reste pyridyle,
R⁴ est un reste alkyle en C₁ à C₄, qui peut être substitué par un radical hydroxy ou (alkoxy en C₁ à C₄)-carbonyle, ou un reste benzyle,
R⁵ est un reste alkyle en C₁ à C₄, qui peut être substitué par un radical hydroxy,
ou bien
R⁴ et R⁵ forment, conjointement avec l'atome d'azote auxquels ils sont liés, un noyau imidazolyle, triazolyle ou tétrazolyle ou un reste de formule 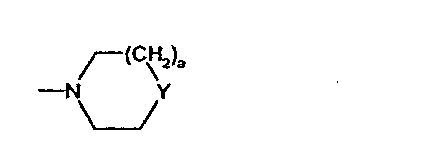
dans laquelle
a a la valeur zéro ou un, et
Y est un groupe CH₂, CH₂CH₂, -O- ou -S- et
R⁶ est un reste pyridyle, qui est substitué une ou deux fois par du fluor, ou un reste thiazolyle,
et leurs sels.

2. Composés suivant la revendication 1, dans lesquels
R⁴ et R⁵ forment, conjointement avec l'atome d'azote auquel ils sont liés, un noyau morpholinyle ou thiomorpholinyle,
et leurs sels.

3. Composés suivant la revendication 1, dans lesquels
R¹, R² représentent indépendamment l'un de l'autre le fluor, le chlore ou le brome,
et leurs sels.

4. Composés suivant la revendication 1, dans lesquels
R¹, R² représentent indépendamment l'un de l'autre le fluor, le chlore ou le brome, et
R⁴ et R⁵ forment un noyau morpholinyle ou thiomorpholinyle conjointement avec l'atome d'azote auquel ils sont liés,
et leurs sels.

5. Composés des exemples 5, 7, 9, 11, 12, 15, 24, 31, 34, 45.

6. Procédé de production des composés suivant les revendications 1 à 5, selon lequel
[A] on fait réagir des composés de formule 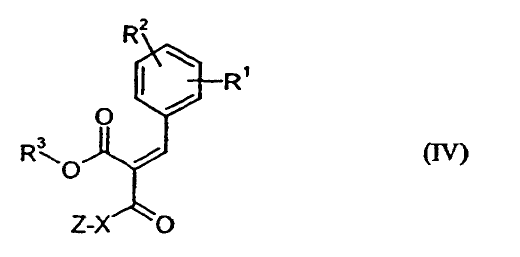 dans laquelle
R¹ à R³, X et Z ont les définitions indiquées dans les revendications 1 à 5,
avec des amidines de formule 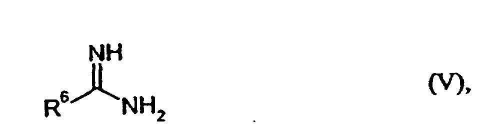 dans laquelle
R⁶ a la définition indiquée dans les revendications 1 à 5,
ou leurs sels,
ou bien
[B] on fait réagir des composés de formule 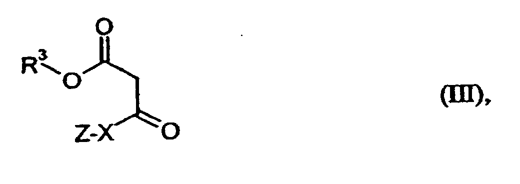 dans laquelle
R³, X et Z ont les définitions indiquées dans les revendications 1 à 5,
en procédant en une seule étape, avec les aldéhydes de formule 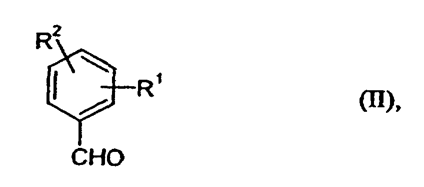 dans laquelle
R¹ et R² ont les définitions indiquées dans les revendications 1 à 5,
avec des amidines de formule 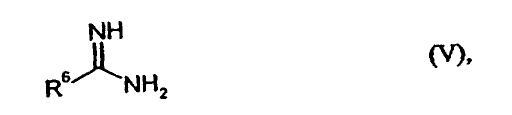 dans laquelle
R⁶ a la définition indiquée dans les revendications 1 à 5,
ou leurs sels, ou aussi,
[C] dans la mesure où X dans la formule (I) représente un groupe méthylène, on fait réagir des composés de formule 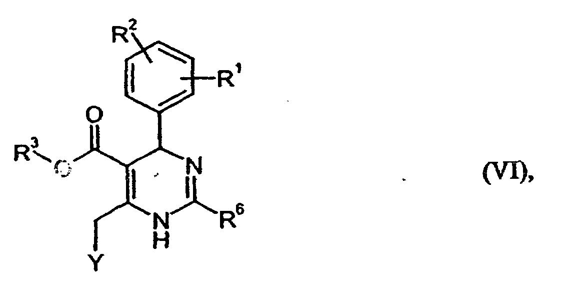 dans laquelle
R¹ à R³ et R⁶ ont les définitions indiquées dans les revendications 1 à 5 et
Y représente un groupe susceptible d'échange nucléophile, avec des composés de formule 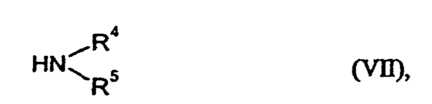
dans laquelle
R⁴ et R⁵ ont les définitions indiquées dans les revendications 1 à 5, ou aussi
[D] dans la mesure où X dans la formule (I) représente un groupe éthylène, on fait réagir des composés de formule 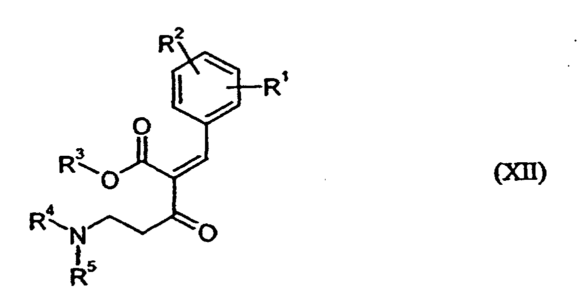
dans laquelle
R¹ à R⁵ ont les définitions indiquées dans la revendication 1
avec des amidines de formule 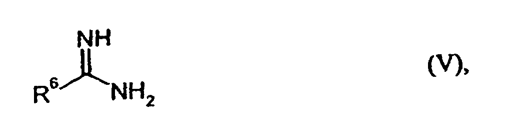 dans laquelle
R⁶ a la définition indiquée dans les revendications 1 à 5,
ou leurs sels.

7. Procédé de production des composés suivant les revendications 1 à 5, selon lequel
[A] on transforme des aldéhydes de formule 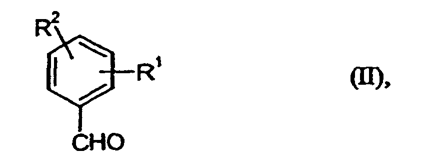 dans laquelle
R¹ et R² ont les définitions indiquées dans les revendications 1 à 5,
tout d'abord avec des β-cétoesters de formule 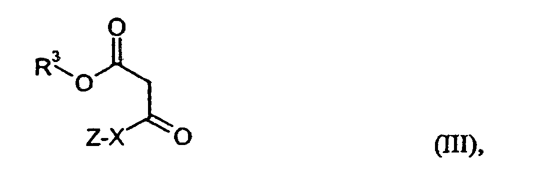 dans laquelle
R³, X et Z ont les définitions indiquées dans les revendications 1 à 5,
en composés benzylidéniques de formule 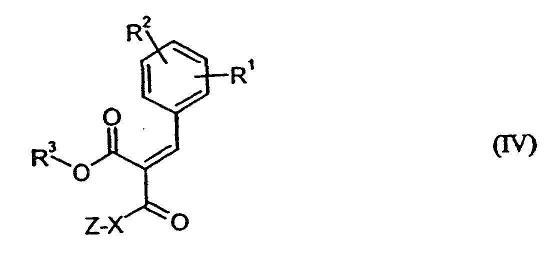 puis on fait réagir ces composés avec des amidines de formule 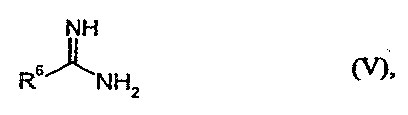 dans laquelle
R⁶ a la définition indiquée dans les revendications 1 à 5,
ou leurs sels, ou bien
[B] on fait réagir des composés de formule (III) en procédant en une seule étape avec des aldéhydes (II) et des amidines (V) ou leurs sels, ou aussi
[C] dans la mesure où X dans la formule (I) représente un groupe méthylène, on fait réagir des composés de formule 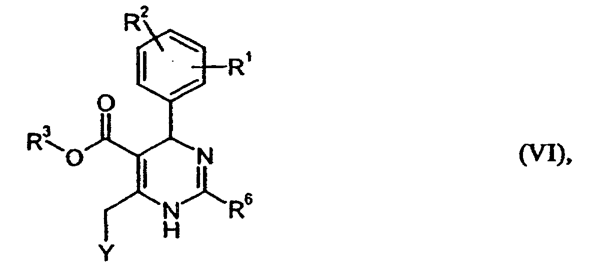 dans laquelle
R¹ à R³ et R⁶ ont les définitions indiquées dans les revendications 1 à 6 et
Y est un groupe susceptible d'échange nucléophile
avec des composés de formule 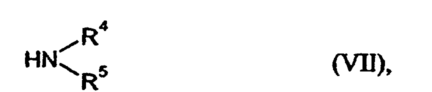 dans laquelle
R⁴ et R⁵ ont les définitions indiquées dans les revendications 1 à 5, ou aussi
[D] dans la mesure où X dans la formule (I) représente un groupe éthylène, on transforme des composés de formule 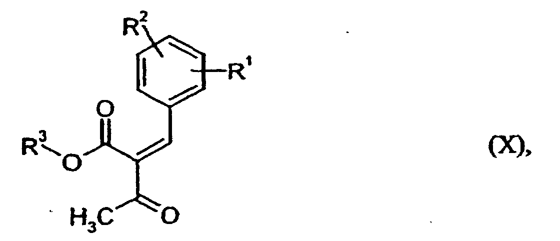
dans laquelle
R¹ à R³ ont les définitions indiquées dans les revendications 1 à 5,
avec des sels d'immonium de formule 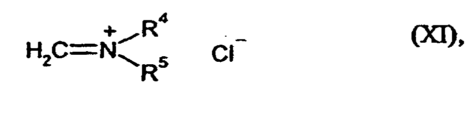 dans laquelle
R⁴ et R⁵ ont les définitions indiquées dans les revendications 1 à 5,
en composés de formule 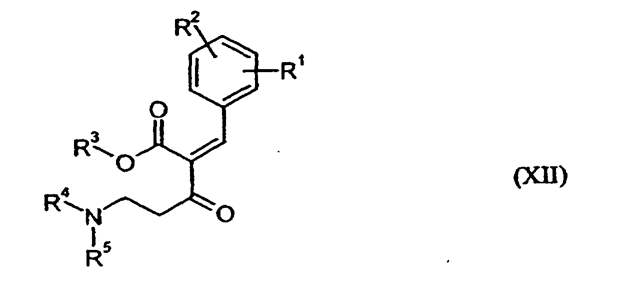 puis on fait réagir ces composés avec des amidines de formule (V) ou leurs sels.

8. Composés de formule (V) dans laquelle 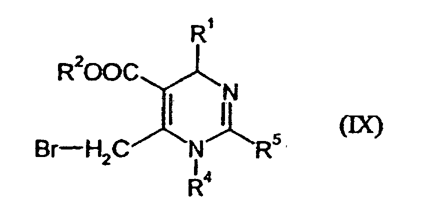
R¹, R², R⁴ et R⁵ ont les définitions indiquées dans les revendications 1 à 5.

9. Composés suivant les revendications 1 à 5, destinés à combattre des maladies.

10. Médicament, contenant au moins un composé suivant les revendications 1 à 5 et, le cas échéant, d'autres substances pharmaceutiques actives.

11. Utilisation de composés des revendications 1 à 5 pour la préparation d'un médicament destiné au traitement et à la prophylaxie de maladies virages.

12. Utilisation de composés des revendications 1 à 5 pour la préparation d'un médicament destiné au traitement et à la prophylaxie d'infections à virus de l'hépatite B.

13. Combinaisons
A) d'au moins une dihydropyrimidine suivant les revendications 1 à 5,
B) d'au moins un agent anti-VHB différent de A, et le cas échéant,
C) d'au moins un immunomodulateur.

14. Combinaisons suivant la revendication 13, dans lesquelles le composant B est un inhibiteur de VHB-polymérase.

15. Combinaisons suivant la revendication 13, dans lesquelles le composant B est la lamivudine.

16. Combinaisons suivant la revendication 13, dans lesquelles le composant B est choisi parmi les composés de formule 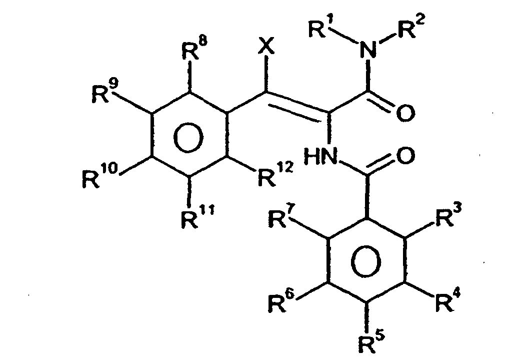 dans laquelle
R¹ et R² représentent indépendamment l'un de l'autre un reste alkyle en C₁ à C₄ ou forment, conjointement avec l'atome d'azote auquel ils sont liés, un noyau de 5 à 6 atomes, qui comprennent du carbone et/ou de l'oxygène,
R³-R¹² représentent indépendamment les uns des autres l'hydrogène, un halogène, un reste alkyle en C₁ à C₄, un reste alkoxy en C₁ à C₄ éventuellement substitué, un, groupe nitro, cyano ou trifluorométhyle,
R¹³ représente l'hydrogène, un reste alkyle en C₁ à C₄, acyle en C₁ à C₇ ou aralkyle, et
X est un halogène ou un reste alkyle en C₁ à C₄ éventuellement substitué,
et leurs sels.

17. Combinaisons suivant la revendication 16, dans lesquelles
X représente le chlore, A est un reste 1-pipérydinyle et Y et Z représentent chacun un reste phényle.

18. Combinaisons suivant les revendications 13 à 17, dans lesquelles l'immunomodulateur C contient des interférons.

19. Combinaisons A) de dihydropyrimidyne suivant les revendications 1 à 5, B) de lamivudine et le cas échéant C) d'interféron.

20. Procédé de préparation des combinaisons suivant les revendications 13 à 19, **caractérisé en ce qu'**on combine ou réunit d'une manière convenable les composés A, B et le cas échéant C.

21. Combinaisons suivant les revendications 13 à 19, destinées à combattre des maladies.

22. Médicaments contenant au moins une combinaison suivant les revendications 13 à 19 et, le cas échéant, d'autres substances pharmaceutiques actives.

23. Utilisation de combinaisons suivant les revendications 13 à 19 pour la préparation d'un médicament destiné au traitement et à la prophylaxie de maladies virales.

24. Utilisation de combinaisons suivant les revendications 13 à 19 pour la préparation d'un médicament destiné au traitement et à la prophylaxie d'infections à virus de l'hépatite B.
